# EUROPEAN PATENT APPLICATION

(11) **EP 4 306 127 A1**
(43) Date of publication of application: **17.01.2024**
(21) Application number: 22767260.7
(22) Date of filing: 11.03.2022
(51) Int. Cl.: A61K 39/395, A61P 21/04, C07K 16/28, C12N 15/13

(54) **PHARMACEUTICAL COMPOSITION FOR TREATMENT OR PREVENTION OF MYASTHENIA GRAVIS**

(30) Priority: 12.03.2021 JP 2021040206
(71) Applicant: CHUGAI SEIYAKU KABUSHIKI KAISHA, Tokyo, 115-8543 (JP); F. Hoffmann-La Roche AG, 4070 Basel (CH)
(72) Inventor: OZAWA, Takatoshi, Tokyo 103-8324 (JP); ZHOU, Mandi, Tokyo 103-8324 (JP); ITO, Hajime, Tokyo 103-8324 (JP); YOSHIDA, Shunsuke, Tokyo 103-8324 (JP); SMITH, Jillian, Welwyn Garden City AL71TW (GB); LENNON-CHRIMES, Sian, Welwyn Garden City, AL71TW (GB); KRUMOVA, Petranka, 4070 Basel (CH); SILBER BAUMANN, Hanna, 4070 Basel (CH)
(74) Representative: Vossius & Partner Patentanwälte Rechtsanwälte mbB
(86) International application number: PCT/JP2022/010791
(87) International publication number: WO 2022/191306

(57) **Abstract**

The present disclosure provides pharmaceutical compositions for treating or preventing myasthenia gravis comprising satralizumab as an active ingredient.

## Description

### [Technical Field]

The present invention relates to pharmaceutical compositions for use in treatment or prevention of myasthenia gravis.

### [Background Art]

Myasthenia gravis (MG) is an autoimmune disease in which receptors on the muscle side of neuromuscular junctions are destroyed by autoantibodies. Currently there is no therapy for complete cure of the disease, and in Japan, it is designated as a designated intractable disease (NPL1). Existing therapies for generalized myasthenia gravis (generalized MG, gMG) are based on drug treatment (NPL1).

Therapeutic drugs currently used for long-term treatment of MG, such as steroids and immunosuppressants, may cause various side effects including deterioration in physical appearance, osteoporosis, impaired glucose tolerance, diarrhea, muscle spasm, and infections. In addition, as many of the existing therapeutic drugs have not undergone randomized controlled trials to test their efficacy on MG patients, their evidence is limited. For intravenous immunoglobulin therapy (IVIg) and blood purification/replacement therapy, their effects are temporary, and therefore regular treatment including hospitalization is required for long-term control of the symptoms, which may place huge physical, mental, and economic burdens on patients. Therefore, there is a high unmet need for treatment or prevention of MG.

Recently, the biological agent eculizumab has become covered by insurance for anti-acetylcholine receptor (AChR) antibody-positive generalized MG patients only when the symptoms are difficult to control by IVIg or blood purification therapy. However, it has not been actively prescribed because at present it is not indicated for anti-muscle-specific tyrosine kinase (MuSK) antibody-positive MG, anti-low-density lipoprotein (LDL) receptor related protein 4 (Lrp4) antibody-positive MG, or autoantibody-negative MG, and moreover, it requires vaccination to prevent meningococcal infection, and is very expensive.

Accordingly, both in and outside Japan, there is a demand for a new treatment or prevention with established efficacy and safety for MG.

In studies of MG, increased IL-6 levels have been observed in the blood and muscle tissues of non-clinical animal models and MG patients, and the administration of anti-IL-6 antibodies has been shown to be therapeutically effective on MG rats (NPL2 to NPL8), suggesting the possible involvement of IL-6 in the pathological mechanism of MG. Tocilizumab, a blocker of IL-6 signaling, has also been shown to be effective in two patients with moderate and severe AChR antibody-positive MG and insufficient response to rituximab (NPL2). Moreover, it has also been shown that IL-6 antagonists are beneficial to the treatment of immune disorders (PTL1). Thus, the inhibition of IL-6 signaling may serve as a therapeutic option for MG patients.

Humanized antibodies like tocilizumab are first-generation antibody drugs. By improving first-generation antibody drugs, second-generation antibody drugs with improved efficacy, convenience, and cost are being developed. Among the second-generation antibody drugs is satralizumab (SA237), which is a novel anti-IL-6 receptor antibody to which improvement technologies such as enhancement of antigen-binding ability, pharmacokinetics, and stability, and reduction of immunogenicity risk, have been applied (PTL2 and PTL3).

Satralizumab is a pH-dependent binding humanized anti-IL-6 receptor monoclonal antibody. It specifically targets the human IL-6 receptor (IL-6R), and suppresses IL-6 signaling by inhibiting the binding of IL-6 to membrane-bound IL-6R and soluble IL-6R. Satralizumab was constructed by modifying the amino acid sequence of tocilizumab to prolong its plasma half-life. Satralizumab also has pH-dependent binding characteristics to its antigen, IL-6R, and shows a decreased antibody molecule isoelectric point and stronger binding to FcRn. Moreover, its Fc region has been modified to minimize the antibody-dependent cellular cytotoxicity and complement-dependent cytotoxic effector activity.

Prior-art literature information related to the invention of the present application is shown below.

### [Citation List]

### [Non-Patent Literature]

[NPL1] Practical Guideline for Myasthenia Gravis (MG) 2014 (editorial supervisor: Societas Neurologica Japonica), Nankodo Co., Ltd.
[NPL2] D I Jonsson, et al., Beneficial effect of tocilizumab in myasthenia gravis refractory to rituximab. Neuromuscular Disorders 27 (2017) 565-568
[NPL3] Deng C, Goluszko E, Tuzun E, et al., Resistance to experimental autoimmune myasthenia gravis in IL-6-deficient mice is associated with reduced germinal center formation and C3 production. J Immunol. 2002;169(2):1077-83.
[NPL4] Hu Y, Wang J, Rao J, et al., Comparison of peripheral blood B cell subset ratios and B cell-related cytokine levels between ocular and generalized myasthenia gravis. International Immunopharmacology 2020;80:106130.
[NPL5] Zhang CJ, et al., Augmentation of Circulating Follicular Helper T Cells and Their Impact on Autoreactive B Cells in Myasthenia Gravis. J Immunol. 2016 Oct 1;197(7):2610-7.
[NPL6] Mocchegiani E, et al., Different age-related effects of thymectomy in myasthenia gravis: role of thymoma, zinc, thymulin, IL-2 and IL-6. Mech Ageing Dev. 2000 Aug 15;117(1-3):79-91.
[NPL7] Maurer, M., Bougoin, S., Feferman, T. et al., IL-6 and Akt are involved in muscular pathogenesis in myasthenia gravis. acta neuropathol commun 3, 1 (2015).
[NPL8] Miriam C. Souroujon et al., Regulatory T cell-based immunotherapies in experimental autoimmune myasthenia gravis. Annals of the New York Academy of Science, 2012 1274 120-126.

### [Patent Literature]

[PTL1] WO2005/028514
[PTL2] WO2010/035769
[PTL3] WO2016/136933

### [Summary of Invention]

### [Technical Problem]

Satralizumab has a mechanism of action different from that of existing therapeutic drugs for MG. Specifically, satralizumab specifically targets human IL-6R, and suppresses IL-6 signaling by blocking the binding of IL-6 to membrane-bound and soluble IL-6R (sIL-6R). It is expected to be a new therapeutic option for MG.

The present invention has been made in view of these circumstances. An objective of the present invention is to apply satralizumab, which is an anti-IL-6 receptor antibody as a second-generation antibody drug to which improvement technologies such as enhancement of antigen-binding ability, pharmacokinetics, and stability, and reduction of immunogenicity risk, have been applied, and which is also an antibody with a mechanism of action different from that of existing therapeutic drugs, i.e. suppressing IL-6 signaling, to the treatment or prevention of MG.

### [Solution to Problem]

To solve the above-mentioned problem, the present inventors focused on the effect of the second-generation antibody drug satralizumab in suppressing IL-6 signaling, and discovered that it can be used for treating MG, thereby completing the present invention.

The present invention specifically includes the following:
[1] A pharmaceutical composition for treatment or prevention for a patient with myasthenia gravis comprising, as an active ingredient:
   (i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
   (ii) an antibody comprising a heavy chain variable region comprising the amino acid sequence of SEQ ID NO: 1 and a light chain variable region comprising the amino acid sequence of SEQ ID NO: 2; or
   (iii) an antibody comprising a heavy chain comprising the amino acid sequence of SEQ ID NO: 3 and a light chain comprising the amino acid sequence of SEQ ID NO: 4,
   wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive.
[2] The pharmaceutical composition of [1], wherein the antibody is satralizumab.
[3] The pharmaceutical composition of [1] or [2], wherein the patient with myasthenia gravis is anti-MuSK antibody-positive or anti-Lrp4 antibody-positive.
[4] The pharmaceutical composition of any one of [1] to [3], wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.
[5] The pharmaceutical composition of any one of [1] to [3], wherein the patient with myasthenia gravis is a patient with a total MG Activities of Daily Living (MG-ADL) score of 5 or higher, and wherein half or more of the score is related to non-ocular symptoms.
[6] The pharmaceutical composition of any one of [1] to [3], wherein the myasthenia gravis is generalized myasthenia gravis.
[7] The pharmaceutical composition of any one of [1] to [3], which reduces the MG-ADL score.
[8] The pharmaceutical composition of any one of [1] to [3], which reduces the Quantitative Myasthenia Gravis (QMG) score, 15-item Myasthenia Gravis (MG) Quality of Life scale (revised)(MG-QOL 15r) score, Neurology Quality-of-Life Fatigue Short Form (Neuro-QOL Fatigue) score, or Myasthenia Gravis Composite (MGC) score.
[9] The pharmaceutical composition of any one of [1] to [8], wherein the dose of the antibody for a patient with a body weight of 100 kg or less is 120 mg/administration, and the dose of the antibody for a patient with a body weight of more than 100 kg is 180 mg/administration.
[10] The pharmaceutical composition of any one of [1] to [8], wherein the dose of the antibody for a patient with a body weight of 100 kg or less is 120 mg/administration, and the dose of the antibody for a patient with a body weight of more than 100 kg is 240 mg/administration.
[11] The pharmaceutical composition of any one of [1] to [8], wherein the dose of the antibody for a patient with a body weight of 100 kg or less is 180 mg/administration, and the dose of the antibody for a patient with a body weight of more than 100 kg is 240 mg/ administration.
[12] The pharmaceutical composition of any one of [1] to [11], wherein the composition is administered at a standard dosing interval after a short-interval dosing period during which the composition is administered at the same dose as a standard dose multiple times at a dosing interval that is shorter than the standard dosing interval.
[13] An agent for treatment or prevention for a patient with myasthenia gravis, comprising as an active ingredient an antibody comprising the following, wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive:
   (i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
   (ii) an antibody comprising a heavy chain variable region comprising the amino acid sequence of SEQ ID NO: 1 and a light chain variable region comprising the amino acid sequence of SEQ ID NO: 2; or
   (iii) an antibody comprising a heavy chain comprising the amino acid sequence of SEQ ID NO: 3 and a light chain comprising the amino acid sequence of SEQ ID NO: 4.
[14] The agent of [13], wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.
[15] The agent of [13] or [14], wherein the dose of the antibody for a patient with a body weight of 100 kg or less is 120 mg/administration, and the dose of the antibody for a patient with a body weight of more than 100 kg is 180 mg/administration.
[16] The agent of [13] or [14], wherein the dose of the antibody for a patient with a body weight of 100 kg or less is 120 mg/administration, and the dose of the antibody for a patient with a body weight of more than 100 kg is 240 mg/administration.
[17] The agent of [13] or [14], wherein the dosage of the antibody for a patient with a body weight of 100 kg or less is 180 mg/administration, and the dosage of the antibody for a patient with a body weight of more than 100 kg is 240 mg/administration.
[18] A method for treating or preventing myasthenia gravis, comprising administering an antibody comprising the following to a patient in need thereof, wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive:
   (i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
   (ii) an antibody comprising a heavy chain variable region comprising the amino acid sequence of SEQ ID NO: 1 and a light chain variable region comprising the amino acid sequence of SEQ ID NO: 2; or
   (iii) an antibody comprising a heavy chain comprising the amino acid sequence of SEQ ID NO: 3 and a light chain comprising the amino acid sequence of SEQ ID NO: 4.
[19] The method of [18], wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.
[20] The method of [18] or [19], wherein the dose of the antibody for a patient with a body weight of 100 kg or less is 120 mg/administration, and the dose of the antibody for a patient with a body weight of more than 100 kg is 180 mg/administration.
[21] The method of [18] or [19], wherein the dose of the antibody for a patient with a body weight of 100 kg or less is 120 mg/administration, and the dose of the antibody for a patient with a body weight of more than 100 kg is 240 mg/administration.
[22] The method of [18] or [19], wherein the dose of the antibody for a patient with a body weight of 100 kg or less is 180 mg/administration, and the dose of the antibody for a patient with a body weight of more than 100 kg is 240 mg/administration.
[23] Use of an antibody comprising the following in the manufacture of an agent for treating or preventing myasthenia gravis, wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive:
   (i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
   (ii) an antibody comprising a heavy chain variable region comprising the amino acid sequence of SEQ ID NO: 1 and a light chain variable region comprising the amino acid sequence of SEQ ID NO: 2; or
   (iii) an antibody comprising a heavy chain comprising the amino acid sequence of SEQ ID NO: 3 and a light chain comprising the amino acid sequence of SEQ ID NO: 4.
[24] The use of [23], wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.
[25] The use of [23] or [24], wherein the dose of the antibody for a patient with a body weight of 100 kg or less is 120 mg/administration, and the dose of the antibody for a patient with a body weight of more than 100 kg is 180 mg/administration.
[26] The use of [23] or [24], wherein the dose of the antibody for a patient with a body weight of 100 kg or less is 120 mg/administration, and the dose of the antibody for a patient with a body weight of more than 100 kg is 240 mg/administration.
[27] The use of [23] or [24], wherein the dose of the antibody for a patient with a body weight of 100 kg or less is 180 mg/administration, and the dose of the antibody for a patient with a body weight of more than 100 kg is 240 mg/administration.
[28] An antibody for use in treatment or prevention for a patient with myasthenia gravis, wherein the antibody comprises the following, and wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive:
   (i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
   (ii) an antibody comprising a heavy chain variable region comprising the amino acid sequence of SEQ ID NO: 1 and a light chain variable region comprising the amino acid sequence of SEQ ID NO: 2; or
   (iii) an antibody comprising a heavy chain comprising the amino acid sequence of SEQ ID NO: 3 and a light chain comprising the amino acid sequence of SEQ ID NO: 4.
[29] The antibody of [28], wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.
[30] The antibody of [28] or [29], wherein the dose of the antibody for a patient with a body weight of 100 kg or less is 120 mg/administration, and the dose of the antibody for a patient with a body weight of more than 100 kg is 180 mg/administration.
[31] The antibody of [28] or [29], wherein the dose of the antibody for a patient with a body weight of 100 kg or less is 120 mg/administration, and the dose of the antibody for a patient with a body weight of more than 100 kg is 240 mg/administration.
[32] The antibody of [28] or [29], wherein the dose of the antibody for a patient with a body weight of 100 kg or less is 180 mg/administration, and the dose of the antibody for a patient with a body weight of more than 100 kg is 240 mg/administration.
[33] A medicament comprising a fixed dose of 180 mg of satralizumab as an active ingredient.
[34] A medicament comprising a fixed dose of 240 mg of satralizumab as an active ingredient.
[35] An article of manufacture comprising a fixed dose of 180 mg of satralizumab in a pharmaceutically acceptable excipient.
[36] An article of manufacture comprising a fixed dose of 240 mg of satralizumab in a pharmaceutically acceptable excipient.
[37] An article of manufacture comprising:
   (i) a container;
   (ii) a pharmaceutical composition comprising a fixed dose of 180 mg of satralizumab in the container; and
   (iii) optionally, a label on or a package insert accompanying the container.
[38] An article of manufacture comprising:
   (i) a container;
   (ii) a pharmaceutical composition comprising a fixed dose of 240 mg of satralizumab in the container; and
   (iii) optionally, a label on or a package insert accompanying the container.
[39] The article of manufacture of any one of [35]-[38], wherein the article of manufacture is a subcutaneous administration device.
[40] The article of manufacture of any one of [35]-[38], wherein the article of manufacture is a prefilled syringe.
[41] The article of manufacture of any one of [35]-[38], wherein the article of manufacture is an autoinjector.

### Effects of the Invention

The present invention can provide pharmaceutical compositions for treating or preventing myasthenia gravis with a mechanism of action different from that of existing therapeutic drugs.

### [Brief Description of Drawings]

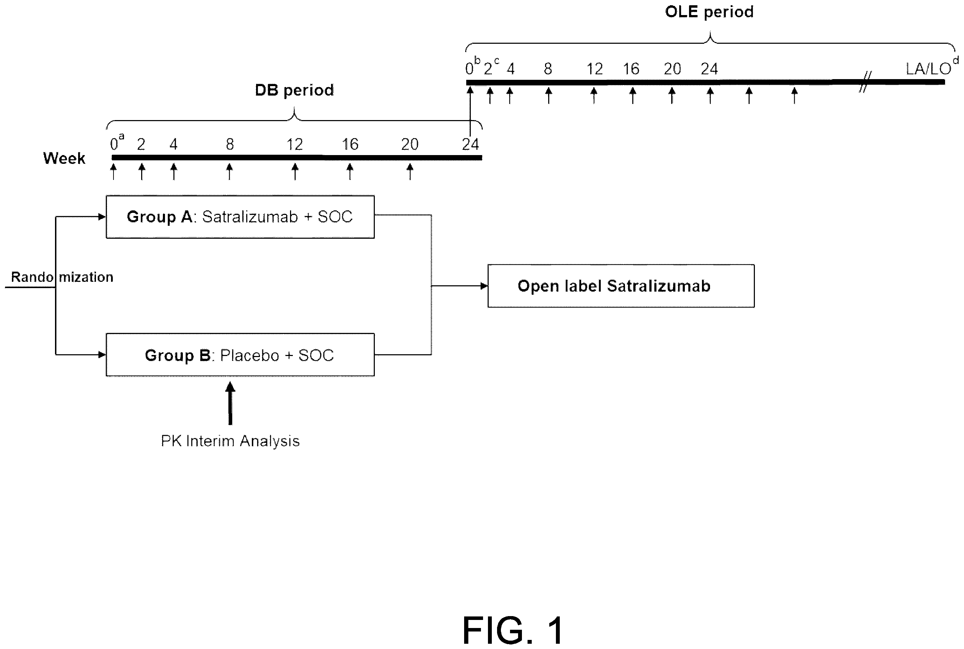

Figure 1 shows the study design of this Phase III, randomized, double-blinded, placebo-controlled, multicenter study. DB represents double blind, LA represents last assessment, LO represents last observation, OLE represents open-label extension, PK represents pharmacokinetics, and SOC represents standard of care. a: Week 0 baseline assessments will be collected pre-dose. b: Week 0 of OLE period coincides with Week 24 of DB period, c: Patients treated with active drug in DB period will be administered a placebo dose at Week 2 of the OLE period to maintain blinding of treatment assignment in the DB period. d: The total length of study, from screening of first patient to the end of OLE period, is estimated to be approximately 4 years.

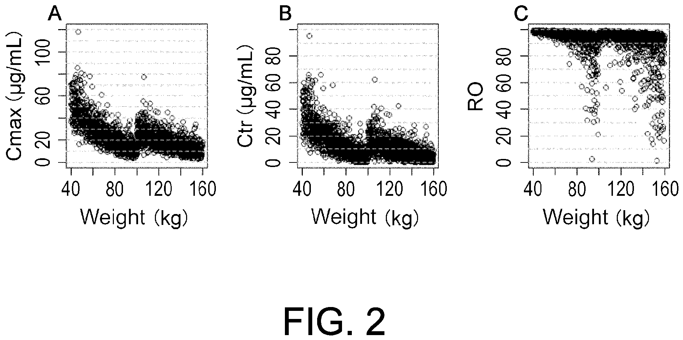

Figure 2 shows the predicted steady-state exposure parameters (maximum concentration (Cₘₐₓ), trough concentration (C_{trough})) and receptor occupancy (RO) values in serum following administration of 120 mg and 180 mg every 4 weeks in patients weighing 100 kg or less (40-100 kg) and patients weighing more than 100 kg (100-160 kg), respectively. Cₘₐₓ represents steady-state maximum concentration, Cₜᵣ represents steady-state trough concentration, and RO represents steady-state receptor occupancy. Plots show the results of simulation for 2000 individuals. Cₘₐₓ predicted values are shown in Figure 2A, C_{trough} predicted values in Figure 2B, and RO predicted values in Figure 2C. Points are simulated data based on an assumption that the percentage of patients who are ADA (anti-drug antibody)-positive is the same as that seen in NMOSD (neuromyelitis optica spectrum disorder) studies. Dotted horizontal lines have been added for reference.
Figure 3 shows that a dosing regimen of 180 mg and 240 mg every 4 weeks for patients weighing 100 kg or less (40-100 kg) and more than 100 kg (100-160 kg), respectively, would be expected to maintain the target level of RO across the body weight ranges. Cₜᵣ represents steady-state trough concentration, and RO represents steady-state receptor occupancy.

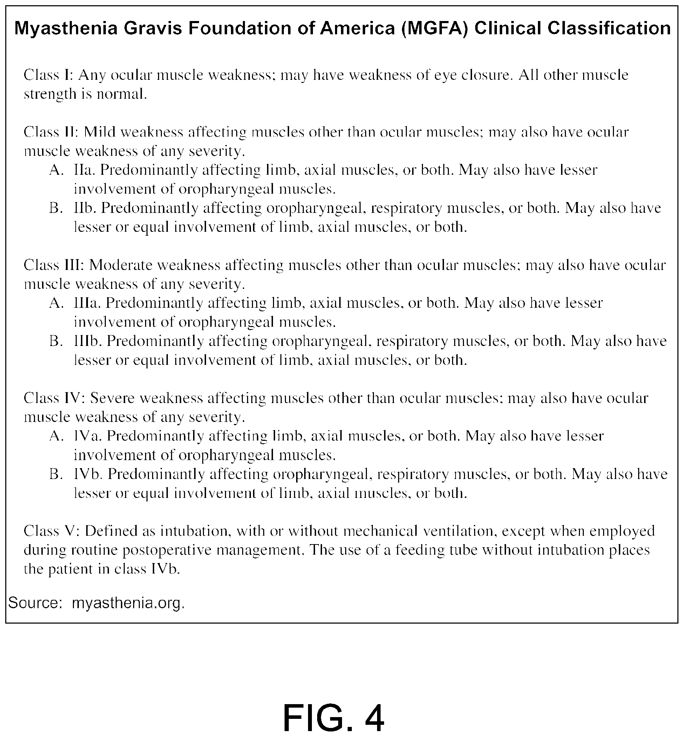

Figure 4 shows the MGFA (MG Foundation of America) classification system.
Figure 5 shows a sample of the MG Activities of Daily Living (MG-ADL) questionnaire.
Figure 6 shows a sample of the Quantitative Myasthenia Gravis (QMG) questionnaire.
Figure 7 shows a sample of the Myasthenia Gravis Composite (MGC) questionnaire.
Figure 8 shows a sample of the 15-item Myasthenia Gravis (MG) Quality of Life scale (revised) (MG-QOL 15r) questionnaire.
Figure 9 shows a sample of the Neurology Quality-of-Life Fatigue (Neuro-QoL Fatigue) Short Form scale.
Figure 10 shows the fractions of patients with a clearance outside the range of 0.8-1.25 of the population mean and patients with an RSE of more than 0.2, for each number of assessed cases, in simulations performed for HV (healthy adults) and NMOSD (neuromyelitis optica spectrum disorder) patients. RSE represents residual standard error.

### [Description of Embodiments]

Herein below, the present invention will be described in detail.

The present invention relates to pharmaceutical compositions for use in the treatment or prevention of myasthenia gravis (MG).

An "antibody" in the present invention is an antibody that blocks signal transduction by IL-6 and inhibits the biological activities of IL-6. An antibody is preferably an antibody that has an inhibitory effect against the binding of IL-6, IL-6 receptor, or gp130.

Antibodies in the present invention include, but are not particularly limited to, for example, anti-IL-6 antibodies, anti-IL-6 receptor antibodies, and anti-gp130 antibodies. Preferred antibodies in the present invention include anti-IL-6 receptor antibodies that recognize an IL-6 receptor.

An anti-IL-6 receptor antibody used in the present invention can be obtained as either a polyclonal or monoclonal antibody using known methods. In particular, an anti-IL-6 receptor antibody used in the present invention is preferably a monoclonal antibody derived from a mammal. Monoclonal antibodies derived from a mammal include those produced by a hybridoma and those produced by a host that has been transformed with an expression vector containing an antibody gene using genetic engineering methods. By binding to an IL-6 receptor, this antibody inhibits the binding of IL-6 to an IL-6 receptor, and blocks transduction of the biological activity of IL-6 into cells.

Examples of such an antibody include the MR16-1 antibody (Tamura, T. et al. Proc. Natl. Acad. Sci. USA (1993) 90, 11924-11928), PM-1 antibody (Hirata, Y. et al., J. Immunol. (1989) 143, 2900-2906), AUK12-20 antibody, AUK64-7 antibody, and AUK146-15 antibody (International Patent Application Publication No. WO 92-19759). Among them, the PM-1 antibody is an example of a preferred monoclonal antibody against the human IL-6 receptor, and the MR16-1 antibody is an example of a preferred monoclonal antibody against the mouse IL-6 receptor.

Basically, hybridomas that produce an anti-IL-6 receptor monoclonal antibody can be produced using known techniques as follows: an IL-6 receptor is used as a sensitizing antigen to perform immunization by a conventional immunization method, the resulting immune cells are fused with known parent cells by a conventional cell fusion method, and then the cells are screened for monoclonal antibody-producing cells by a conventional screening method.

Specifically, anti-IL-6 receptor antibodies can be produced as below. A human IL-6 receptor or mouse IL-6 receptor to be used as a sensitizing antigen for obtaining antibodies can be obtained by, for example, using the IL-6 receptor gene and/or amino acid sequences respectively disclosed in European Patent Application Publication No. EP 325474 and Japanese Patent Application *Kokai* Publication No. (JP-A) H03-155795.

There are two types of IL-6 receptor proteins: one expressed on the cell membrane and the other separated from the cell membrane (soluble IL-6 receptor) (Yasukawa, K. et al., J. Biochem. (1990) 108, 673-676). The soluble IL-6 receptor is essentially composed of the extracellular region of the cell membrane-bound IL-6 receptor, and differs from the membrane-bound IL-6 receptor in that it lacks the transmembrane region or both the transmembrane and intracellular regions. Any IL-6 receptor may be employed as the IL-6 receptor protein, as long as it can be used as a sensitizing antigen for producing an anti-IL-6 receptor antibody to be used in the present invention.

An IL-6 receptor gene sequence is inserted into a known expression vector system and an appropriate host cell is transformed. Then the target IL-6 receptor protein is purified from the inside of the host cell or from the culture supernatant using a known method. This purified IL-6 receptor protein may be used as a sensitizing antigen. Alternatively, a cell expressing the IL-6 receptor or a fusion protein of the IL-6 receptor protein with another protein may be used as a sensitizing antigen.

Mammals to be immunized with a sensitizing antigen are not particularly limited, but are preferably selected in consideration of the compatibility with parent cells used for cell fusion. Typically, rodents such as mice, rats, and hamsters are used.

Animals are immunized with a sensitizing antigen according to known methods. Typically, immunization is performed by, for example, intraperitoneal or subcutaneous injection of the sensitizing antigen to a mammal. Specifically, it is preferable to dilute or suspend the sensitizing antigen in phosphate-buffered saline (PBS), physiological saline, and such, to an appropriate volume, and mix it with an appropriate amount of a conventional adjuvant such as Freund's complete adjuvant if desired and emulsify, and then administer to the mammal every four to 21 days for several times. An appropriate carrier may also be used for immunization with the sensitizing antigen.

After immunizing the mammal in this manner, and confirming that the serum level of a desired antibody has increased, immunized cells are removed from the mammal and subjected to cell fusion. Spleen cells are particularly preferred as the immunized cells to be subjected to cell fusion.

Myeloma cells from mammals are used as parent cells to be fused with the immunized cells. So far, various known cell lines such as P3X63Ag8.653 (Kearney, J. F. et al., J. Immunol (1979) 123, 1548-1550), P3X63Ag8U.1 (Current Topics in Microbiology and Immunology (1978) 81, 1-7), NS-1 (Kohler, G. and Milstein, C., Eur. J. Immunol. (1976) 6, 511-519), MPC-11 (Margulies, D. H. et al., Cell (1976) 8, 405-415), SP2/0 (Shulman, M. et al., Nature (1978) 276, 269-270), F0 (de St. Groth, S. F. et al., J. Immunol. Methods (1980) 35, 1-21), S194 (Trowbridge, I. S., J. Exp. Med. (1978) 148, 313-323), and R210 (Galfre, G. et al., Nature (1979) 277, 131-133) are suitably used.

Basically, cell fusion of the aforementioned immune cells with myeloma cells can be performed according to known methods such as the method of Milstein *et al.* (Kohler, G. and Milstein, C., Methods Enzymol. (1981) 73, 3-46).

More specifically, the cell fusion is performed, for example, in a conventional nutrient culture medium in the presence of a cell fusion promoter. For example, polyethylene glycol (PEG) or Sendai virus (HVJ) is used as the fusion promoter, and if desired, an auxiliary agent such as dimethyl sulfoxide can be further added for use in improving the fusion efficiency.

The ratio of immune cells to myeloma cells used is preferably, for example, 1 to 10 immune cells for each myeloma cell. The culture medium used for the cell fusion is, for example, an RPMI1640 or MEM culture medium suitable for the proliferation of the myeloma cell lines. Other conventional culture media used for this type of cell culture can also be used. Furthermore, serum supplements such as fetal calf serum (FCS) can also be used in combination.

For cell fusion, the fusion cells (hybridomas) of interest are formed by thoroughly mixing predetermined amounts of the aforementioned immune cell and myeloma cell in the aforementioned culture medium, adding a PEG solution (for example, a solution of PEG with an average molecular weight of about 1,000 to 6,000) pre-heated to about 37°C, usually at a concentration of 30% to 60% (w/v), and then mixing them. Then, cell fusion agents and such that are unsuitable for the growth of hybridomas can be removed by repeating the operation of sequentially adding an appropriate culture medium and removing the supernatant by centrifugation.

The hybridomas are selected by culturing in a general selection culture medium, for example, the HAT culture medium (a culture medium containing hypoxanthine, aminopterin, and thymidine). Culturing in the HAT culture medium is continued for a sufficient period, generally from several days to several weeks, to kill cells other than the hybridomas of interest (unfused cells). Then, a standard limiting dilution method is performed to screen for and clone hybridomas that produce an antibody of interest.

Besides obtaining the hybridomas by immunizing non-human animals with an antigen, desired human antibodies having a binding activity to a desired antigen or antigen-expressing cell can be obtained by sensitizing a human lymphocyte with a desired antigen protein or antigen-expressing cell *in vitro,* and fusing the sensitized B lymphocyte with a human myeloma cell such as U266 (see, Japanese Patent Application *Kokoku* Publication No. (JP-B) H01-59878). Further, an antigen or antigen-expressing cell may be administered to a transgenic animal having a repertoire of human antibody genes, and then a desired human antibody may be obtained following the aforementioned method (see, International Patent Application Publication Nos. WO 93/12227, WO 92/03918, WO 94/02602, WO 94/25585, WO 96/34096, and WO 96/33735).

The hybridomas prepared as such that produce monoclonal antibodies can be passaged in a conventional culture medium and stored in liquid nitrogen for a long period.

To obtain monoclonal antibodies from the hybridomas, the following methods may be employed: culturing the hybridomas according to conventional methods and obtaining the antibodies as a culture supernatant or proliferating the hybridomas by administering them to a compatible mammal and obtaining the antibodies from ascites; and so on. The former method is suitable for obtaining antibodies with high purity, and the latter is suitable for large-scale antibody production.

For example, hybridomas that produce anti-IL-6 receptor antibodies can be prepared by the method disclosed in JP-A (Kokai) H03-139293. Such a preparation can be carried out by injecting hybridomas that produce PM-1 antibodies into the abdominal cavity of a BALB/c mouse, obtaining ascites, and then purifying the PM-1 antibodies from the ascites; or by culturing the hybridomas in an appropriate medium (such as an RPMI 1640 medium containing 10% fetal bovine serum, and 5% BM-Condimed H1 (Boehringer Mannheim); the hybridoma SFM medium (GIBCO-BRL); or the PFHM-II medium (GIBCO-BRL)) and then purifying the PM-1 antibodies from the culture supernatant.

Recombinant antibodies can be used as the monoclonal antibodies of the present invention, wherein the recombinant antibodies are produced using genetic recombination techniques by cloning an antibody gene from a hybridoma, inserting the gene into an appropriate vector, and then introducing the vector into a host (see, for example, Borrebaeck, C. A. K. and Larrick, J. W., THERAPEUTIC MONOCLONAL ANTIBODIES, Published in the United Kingdom by MACMILLAN PUBLISHERS LTD, 1990).

More specifically, mRNAs coding for antibody variable (V) regions are isolated from cells that produce antibodies of interest, such as hybridomas. mRNAs can be isolated by preparing total RNAs according to known methods, such as the guanidine ultracentrifugation method (Chirgwin, J. M. et al., Biochemistry (1979) 18, 5294-5299) and the AGPC method (Chomczynski, P. et al., Anal. Biochem. (1987) 162, 156-159), and preparing mRNAs using an mRNA Purification Kit (Pharmacia) and such. Alternatively, mRNAs can be directly prepared using the QuickPrep mRNA Purification Kit (Pharmacia).

cDNAs of the antibody V regions are synthesized from the obtained mRNAs using reverse transcriptase. cDNAs may be synthesized using the AMV Reverse Transcriptase First-strand cDNA Synthesis Kit and such. Further, to synthesize and amplify the cDNAs, the 5'-RACE method (Frohman, M. A. et al., Proc. Natl. Acad. Sci. USA (1988) 85, 8998-9002; Belyavsky, A. et al., Nucleic Acids Res. (1989) 17, 2919-2932) using 5'-Ampli FINDER RACE Kit (Clontech) and PCR may be used. A DNA fragment of interest is purified from the obtained PCR products and then ligated with a vector DNA. Then, a recombinant vector is prepared by using the above, and introduced into *Escherichia coli* and such, and then its colonies are selected to prepare a desired recombinant vector. The nucleotide sequence of the DNA of interest is confirmed by a known method such as the dideoxy method.

When a DNA encoding the V region of the antibody of interest is obtained, the DNA is ligated with a DNA encoding the constant region (C region) of a desired antibody, and inserted into an expression vector. Alternatively, a DNA encoding an antibody V region may be inserted into an expression vector comprising a DNA of an antibody C region.

To produce an antibody to be used in the present invention, an antibody gene is inserted into an expression vector such that it is expressed under the control of an expression-regulating region such as an enhancer and promoter, as described below. Then, the antibody can be expressed by transforming a host cell with this expression vector.

In the present invention, artificially modified recombinant antibodies, for example, chimeric antibodies, humanized antibodies, or human antibodies can be used, for example, to reduce heteroantigenicity against humans. These modified antibodies can be prepared using known methods.

A chimeric antibody can be obtained by ligating a DNA encoding an antibody V region obtained as above with a DNA encoding a human antibody C region, inserting it into an expression vector, and introducing the vector into a host to produce the chimeric antibody (see, European Patent Application Publication No. EP 125023; International Patent Application Publication No. WO 92-19759). This known method can be used to obtain chimeric antibodies useful for the present invention.

Humanized antibodies are also referred to as reshaped human antibodies or antibodies made into the human type. They are produced by transplanting the complementarity determining regions (CDRs) of an antibody from a non-human mammal (for example, a mouse) into the CDRs of a human antibody. General methods for this gene recombination are also known (see, European Patent Application Publication No. EP 125023, International Patent Application Publication No. WO 92-19759).

More specifically, DNA sequences designed to ligate the CDRs of a mouse antibody with the framework regions (FRs) of a human antibody are synthesized by PCR from several oligonucleotides produced to contain overlapping portions at their termini. The obtained DNA is ligated with a DNA encoding a human antibody C region and inserted into an expression vector, and the expression vector is introduced into a host to produce the humanized antibody (see, European Patent Application Publication No. EP 239400, International Patent Application Publication No. WO 92-19759).

Human antibody FRs to be ligated *via* the CDRs are selected so that the CDRs form satisfactory antigen binding sites. The amino acid(s) within the framework regions of the antibody variable regions may be substituted as necessary so that the CDRs of the reshaped human antibody form appropriate antigen binding sites (Sato, K. et al., Cancer Res. (1993) 53, 851-856).

Human antibody constant regions (C regions) are used for the chimeric and humanized antibodies. Examples of human antibody C regions include Cy, and for example, Cγ1, Cγ2, Cy3, or Cy4 may be used. Furthermore, to improve the stability of the antibodies or their production, the human antibody C regions may be modified.

Chimeric antibodies are composed of the variable region of an antibody derived from a non-human mammal and the C region derived from a human antibody; and humanized antibodies are composed of the CDRs of an antibody derived from a non-human mammal and the framework regions and C regions derived from a human antibody. Their antigenicity in the human body is reduced, and thus they are useful as antibodies for use in the present invention.

Preferred specific examples of humanized antibodies for use in the present invention include a humanized PM-1 antibody (see, International Patent Application Publication No. WO 92-19759).

Furthermore, in addition to the aforementioned methods for obtaining human antibodies, techniques for obtaining human antibodies by panning using a human antibody library are also known. For example, the variable region of a human antibody can be expressed on a phage surface as a single chain antibody (scFv) by using the phage display method, and antigen-binding phages can then be selected. By analyzing the genes of the selected phages, the DNA sequence encoding the variable region of the human antibody which binds to the antigen can be determined. Once the DNA sequence of an scFv which binds to the antigen is revealed, an appropriate expression vector comprising the sequence can be prepared to obtain a human antibody. These methods are already known, and reference can be made to WO 92/01047, WO 92/20791, WO93/06213, WO 93/11236, WO 93/19172, WO 95/01438, and WO 95/15388.

The antibody gene constructed as described above can be expressed according to known methods. When a mammalian cell is used, the antibody gene can be expressed by using a DNA in which a commonly used effective promoter gene, the antibody gene to be expressed, and a poly A signal on the 3' side (downstream) of the antibody gene are operatively linked together, or by using a vector comprising the DNA. Examples of a promoter/enhancer include the human cytomegalovirus immediate early promoter/enhancer.

Furthermore, other promoters/enhancers that can be used for expressing the antibodies for use in the present invention include viral promoters/enhancers from retroviruses, polyoma viruses, adenoviruses, simian virus 40 (SV40), and such; and mammalian cell-derived promoters/enhancers such as human elongation factor 1α (HEF1α).

The expression can be easily performed, for example, by following the method in Mulligan *et al.* (Mulligan, R. C. et al., Nature (1979) 277, 108-114) when using the SV40 promoter/enhancer, or by following the method in Mizushima *et al.* (Mizushima, S. and Nagata S., Nucleic Acids Res. (1990) 18, 5322) when using the HEF1α promoter/enhancer.

When *E. coli* is used, the antibody gene can be expressed by operatively linking a commonly used effective promoter gene, a signal sequence for antibody secretion, and the antibody gene to be expressed. Examples of the promoter include a lacZ promoter and an araB promoter. A lacZ promoter can be used according to the method of Ward *et al.* (Ward, E. S. et al., Nature (1989) 341, 544-546; Ward, E. S. et al., FASEB J. (1992) 6, 2422-2427); and an araB promoter can be used according to the method of Better *et al.* (Better, M. et al., Science (1988) 240, 1041-1043).

When the antibody is produced into the periplasm of *E. coli,* the pel B signal sequence (Lei, S. P. et al., J. Bacteriol. (1987) 169, 4379-4383) may be used as a signal sequence for antibody secretion. The antibody produced into the periplasm is isolated, and then appropriately refolded the antibody structure to be used (see, for example, WO 96/30394).

As the replication origin, those derived from SV40, polyoma virus, adenovirus, bovine papilloma virus (BPV) and such may be used. In addition, to increase the gene copy number in a host cell system, the expression vector may comprise the aminoglycoside phosphotransferase (APH) gene, thymidine kinase (TK) gene, *E. coli* xanthine-guanine phosphoribosyltransferase (Ecogpt) gene, dihydrofolate reductase (dhfr) gene, and such, as a selection marker.

Any production system may be used to prepare the antibodies for use in the present invention. The production systems for antibody preparation include *in vitro* and *in vivo* production systems. *In vitro* production systems include those using eukaryotic cells or those using prokaryotic cells.

When eukaryotic cells are used, the production systems include those using animal cells, plant cells, or fungal cells. Such animal cells include (1) mammalian cells such as CHO, COS, myeloma, baby hamster kidney (BHK), HeLa, and Vero; (2) amphibian cells such as *Xenopus* oocytes; and (3) insect cells such as sf9, sf21, and Tn5. Known plant cells include cells derived from *Nicotiana tabacum,* which may be cultured in callus. Known fungal cells include yeasts such as *Saccharomyces* (*e.g., Saccharomyces cerevisiae*) and mold fungi such as *Aspergillus (e.g., Aspergillus niger).*

When prokaryotic cells are used, production systems include those using bacterial cells. Known bacterial cells include *E. coli* and *Bacillus subtilis.*

Antibodies can be obtained by introducing the antibody gene of interest into these cells by transformation, and then culturing the transformed cells *in vitro.* Cells are cultured according to known methods. For example, DMEM, MEM, RPMI 1640, or IMDM may be used as the culture medium, and serum supplements such as fetal calf serum (FCS) may be used in combination. Alternatively, cells introduced with the antibody gene may be transferred into the abdominal cavity and such of an animal to produce the antibodies *in vivo.*

Meanwhile, *in vivo* production systems include those using animals or those using plants. When using animals, production systems include those using mammals or insects.

Mammals that can be used include goats, pigs, sheep, mice, and bovines (Vicki Glaser, SPECTRUM Biotechnology Applications, 1993). Further, insects that can be used include silkworms. When using plants, tobacco and such may be used.

An antibody gene is introduced into these animals or plants, and the antibodies are produced in the body of the animals or plants and then recovered. For example, an antibody gene can be prepared as a fusion gene by inserting it into the middle of a gene encoding a protein uniquely produced into milk, such as goat β casein. DNA fragments comprising the fusion gene, which includes the inserted antibody gene, are injected into goat embryos, and the embryos are introduced into female goats. The desired antibodies are obtained from milk produced by transgenic goats born from the goats that received the embryos, or their progenies. When appropriate, the transgenic goats may be given hormones to increase the volume of milk containing the desired antibodies that they produce (Ebert, K. M. et al., Bio/Technology (1994) 12, 699-702).

When silkworms are used, the silkworms are infected with a baculovirus inserted with the antibody gene of interest, and the desired antibodies are obtained from the body fluids of these silkworms (Maeda, S. et al., Nature (1985) 315, 592-594). Moreover, when tobacco is used, the antibody gene of interest is inserted into a plant expression vector such as pMON530, and the vector is introduced into bacteria such as *Agrobacterium tumefaciens.* This bacterium is used to infect tobacco such as *Nicotiana tabacum,* and then the desired antibody is obtained from the leaves of this tobacco (Julian, K.-C. Ma et al., Eur. J. Immunol. (1994) 24, 131-138).

When producing antibodies using *in vitro* or *in vivo* production systems as described above, DNAs encoding an antibody heavy chain (H chain) and light chain (L chain) may be inserted into separate expression vectors, and a host is then co-transformed with the vectors. Alternatively, the H chain-encoding DNA and L chain-encoding DNA may be inserted into a single expression vector for transforming a host (see International Patent Application Publication No. WO 94-11523).

The antibodies used in the present invention may be antibody fragments or modified products thereof, as long as they can be suitably used in the present invention. For example, antibody fragments include Fab, F(ab')2, Fv, and single chain Fv (scFv) in which the Fvs of the H and L chains are linked *via* an appropriate linker.

Specifically, the antibody fragments are produced by treating antibodies with enzymes such as papain or pepsin, or alternatively, by constructing genes encoding these antibody fragments and introducing them into expression vectors, and then expressing the vectors in appropriate host cells (see, for example, Co, M. S. et al., J. Immunol. (1994) 152, 2968-2976; Better, M. & Horwitz, A. H., Methods in Enzymology (1989) 178, 476-496; Plueckthun, A. & Skerra, A., Methods in Enzymology (1989) 178, 497-515; Lamoyi, E., Methods in Enzymology (1989) 121, 652-663; Rousseaux, J. et al., Methods in Enzymology (1989) 121, 663-666; and Bird, R. E. et al., TIBTECH (1991) 9, 132-137).

An scFv can be obtained by linking the H-chain V region and the L-chain V region of an antibody. In this scFv, the H-chain V region and the L-chain V region are linked *via* a linker, preferably *via* a peptide linker (Huston, J. S. et al., Proc. Natl. Acad. Sci. USA (1988) 85, 5879-5883). The V regions of the H and L chains in an scFv may be derived from any of the antibodies described above. Peptide linkers for linking the V regions include, for example, an arbitrary single chain peptide consisting of 12 to 19 amino acid residues.

A DNA encoding an scFv can be obtained by amplifying a DNA portion that encodes the desired amino acid sequence in template sequences with PCR using a primer pair which defines the termini of the portion, wherein a DNA encoding an H chain or an H-chain V region and a DNA encoding an L chain or an L-chain V region of the aforementioned antibodies are used as the templates, and then further amplifying the amplified DNA portion with a DNA that encodes a peptide linker portion and a primer pair that defines both ends of the linker so that it may be linked to each of the H and L chains.

Once an scFv-encoding DNA has been prepared, an expression vector comprising the DNA and a host transformed with the expression vector can be obtained according to conventional methods. In addition, an scFv can be obtained according to conventional methods by using the host.

Similar to the above, the antibody fragments can be produced by obtaining their genes, expressing them, and then using a host. An "antibody" as used herein encompasses such antibody fragments.

Antibodies bound to various molecules such as polyethylene glycol (PEG) may also be used as modified antibodies. An "antibody" as used herein encompasses such modified antibodies. These modified antibodies can be obtained by chemically modifying the obtained antibodies. Such methods are already established in the art.

Antibodies produced and expressed as above can be isolated from the inside or outside of the cells or from the hosts, and then purified to homogeneity. The antibodies for use in the present invention can be isolated and purified by affinity chromatography. Columns used for the affinity chromatography include protein A columns and protein G columns. Carriers used for the protein A columns include HyperD, POROS, and Sepharose F.F. Other methods used for the isolation and/or purification of ordinary proteins may be used without limitation.

For example, the antibodies used for the present invention may be isolated and purified by appropriately selecting and combining chromatographies other than the above-described affinity chromatography, filtration, ultrafiltration, salting-out, dialysis, and such. Examples of chromatographies include ion-exchange chromatography, hydrophobic chromatography, and gel filtration. These chromatographies can be applied to high performance liquid chromatography (HPLC). Alternatively, reverse phase HPLC may be used.

The concentration of the antibodies obtained as above can be determined by absorbance measurement, ELISA, and such. Specifically, when using absorbance measurement, the concentration can be determined by appropriately diluting the antibody solution with PBS(-), measuring its absorbance at 280 nm, and calculating the concentration by using the conversion factor 1.35 OD / 1 mg/ml. Alternatively, when using ELISA, the concentration can be determined as below. Specifically, 100 µl of goat anti-human IgG (TAG) diluted to 1 µg/ml with 0.1 M bicarbonate buffer (pH 9.6) is added to a 96-well plate (Nunc) and incubated overnight at 4°C to immobilize the antibody. After blocking, 100 µl of an appropriately diluted antibody to be used in the present invention or an appropriately diluted sample comprising the antibody, or human IgG (CAPPEL) as a standard is added, and the plate is incubated for one hour at room temperature.

After washing, 100 µl of 5,000 x diluted alkaline phosphatase-labeled anti-human IgG (BIO SOURCE) is added, and the plate is incubated for one hour at room temperature. After another wash, the substrate solution is added, the plate is incubated, and absorbance at 405 nm is measured using Microplate Reader Model 3550 (Bio-Rad) to calculate the concentration of the antibody of interest.

IL-6 is an inflammatory cytokine produced by T cells, monocytes, macrophages, and fibroblasts. As a regulator of B-cell and T-cell functions, IL-6 acts pleiotropically on the immune system through its specific receptor, IL-6R. Increased IL-6 levels have been observed in various inflammatory autoimmune diseases including rheumatoid arthritis (RA), systemic lupus erythematosus (SLE), NMOSD (Icoz S. et al., Int J Neurosci. 2010;120(1):71-5, Uzawa A. et al., J Neurol. 2009;256(12):2082-4, UzawaA. et al., Mult Scler. 2010;16(12):1443-52) and Castleman's disease (Ishihara K and Hirano T. Cytokine & Growth Factor Reviews 2002;13(4-5):357-68). In studies of MG, which also involves autoimmune abnormality, increased IL-6 levels have been observed in the blood and muscle tissues of non-clinical animal models and MG patients. Therefore, IL-6 may be involved in the pathological mechanism of MG. For example, it may (i) stimulate the maturation of B cells into autoantibody-producing cells (promote autoantibody production) and (ii) induce the differentiation of CD4-positive T cells into Th17 pro-inflammatory T cells (induce chronic inflammation). As IL-6 is not only directly involved in the production of autoantibodies but also has autoantibody-independent functions, it may be implicated in the pathogenesis of MG regardless of the presence or absence of autoantibodies.

Regulatory T cells (Treg), which suppress excessive immune response, and pathogenic helper T17 (Th17) cells are two lymphocyte subsets with opposite activities in autoimmune diseases such as MG. IL-6, a pro-inflammatory cytokine, is a potent factor to switch immune responses from the induction of Tregs to pathogenic Th17 cells in vivo. A study in experimental autoimmune myasthenia gravis (EAMG) model and healthy control rats (Aricha R. et al., J Autoimmun. 2011;36(2):135-41) reported that the equilibrium between Treg and Th17 cells was perturbed in the disease. The upregulated Th17 cell-related genes and downregulated Treg-related genes in the EAMG model showed a tendency of recovery after administration of anti-IL-6 antibodies. In addition, administration of anti-IL-6 antibodies for EAMG suppressed the progression of EAMG and reduced the overall IgG antibody titers and B cells. These data indicate the importance of IL-6 as a factor for modulating autoimmune response in MG.

Furthermore, tocilizumab, a blocker of IL-6 signaling, has also been shown to be effective in two patients with moderate and severe AChR antibody-positive MG and insufficient response to rituximab (Jonsson DI. et al., Neuromuscular Disorders 2017;27(6):565-8). Thus, the inhibition of IL-6 signaling serves as a therapeutic option for MG patients.

Preferred examples of an "IL-6 receptor antibody" in the present invention include tocilizumab which is a humanized anti-IL-6 receptor IgG1 antibody, and humanized anti-IL-6 receptor antibodies produced by modifying the variable and constant regions of tocilizumab, specifically, antibodies that comprise heavy-chain CDR1 comprising the amino acid sequence of SEQ ID NO: 5, heavy-chain CDR2 comprising the amino acid sequence of SEQ ID NO: 6, heavy-chain CDR3 comprising the amino acid sequence of SEQ ID NO: 7, light-chain CDR1 comprising the amino acid sequence of SEQ ID NO: 8, light-chain CDR2 comprising the amino acid sequence of SEQ ID NO: 9, and light-chain CDR3 comprising the amino acid sequence of SEQ ID NO: 10. More preferred antibodies include antibodies that comprise a heavy-chain variable region comprising the amino acid sequence of SEQ ID NO: 1 and a light-chain variable region comprising the amino acid sequence of SEQ ID NO: 2. Still more preferred are antibodies that comprise a heavy chain comprising the amino acid sequence of SEQ ID NO: 3 (heavy chain of SA237) and a light chain comprising the amino acid sequence of SEQ ID NO: 4 (light chain of SA237). SA237 (satralizumab) is particularly preferred.

Such antibodies can be obtained according to the methods described in WO2010/035769, WO2010/107108, WO2010/106812, and such. Specifically, antibodies can be produced using genetic recombination techniques known to those skilled in the art, based on the sequence of the above-mentioned IL-6 receptor antibody (see, for example, Borrebaeck CAK and Larrick JW, THERAPEUTIC MONOCLONAL ANTIBODIES, Published in the United Kingdom by MACMILLAN PUBLISHERS LTD, 1990). A recombinant antibody can be obtained by cloning a DNA encoding the antibody from a hybridoma or an antibody-producing cell such as an antibody-producing sensitized lymphocyte, inserting the DNA into an appropriate vector, and introducing the vector into a host (host cell) to produce the antibody.

Such antibodies can be isolated and purified using isolation and purification methods conventionally used for antibody purification, without limitation. For example, the antibodies can be isolated and purified by appropriately selecting and combining column chromatography, filtration, ultrafiltration, salting-out, solvent precipitation, solvent extraction, distillation, immunoprecipitation, SDS-polyacrylamide gel electrophoresis, isoelectric focusing, dialysis, recrystallization, and such.

The antibodies used in the present invention may be conjugate antibodies that are bound to various molecules such as polyethylene glycol (PEG), radioactive substances, and toxins. Such conjugate antibodies can be obtained by chemically modifying the obtained antibodies. Methods for antibody modification have been already established in this field. Accordingly, the term "antibody" in the present invention encompasses such conjugate antibodies.

In Japan, marketing of satralizumab was approved in June 2020, on the indication "prevention of relapses of neuromyelitis optica spectrum disorder (including neuromyelitis optica)". The safety profiles identified during the international joint phase III clinical trials (SA-307JG test and SA-309JG test) targeting a population of patients with neuromyelitis optica spectrum disorder (NMOSD) and/or neuromyelitis optica (NMO) were mostly favorable. No death cases were reported. The percentage of patients who expressed severe adverse events in the satralizumab group was about the same as that in the placebo group. There was no big difference between the two groups on the frequency of adverse events that led to discontinuation of administration of the test drug, or on the frequency of adverse events that led to drug withdrawal. Safety profiles were similar between the SA-309JG test, which was a single-agent test, and the SA-307JG test, which was a combined test with preexisting therapy (oral steroids and/or immunosuppressive agents).

Myasthenia gravis (MG) is an autoimmune disease caused by impaired stimulus transmission at the neuromuscular junction, due to disruption of the receptors on the muscle side by autoantibodies at the neuromuscular junction.

As it stands, there is no therapy for complete cure and it is designated as a designated intractable disease in Japan. The disease is characterized by indolent fatigable weakness in skeletal muscles, and it worsens by repeating exercise and improves by resting (Practical Guideline for Myasthenia Gravis (MG) 2014 (editorial supervisor: Societas Neurologica Japonica), Nankodo Co., Ltd.). Permanent muscle damage rarely occurs and maximal strength is often good. Meanwhile, decrease in muscular force varies among individual muscles and muscle groups. According to statistics in Japan (Practical Guideline for Myasthenia Gravis (MG) 2014 (editorial supervisor: Societas Neurologica Japonica), Nankodo Co., Ltd.), 71.9% of the patients experience drooping of the eyelid (ptosis) and 47.3% experience double vision (diplopia) as initial symptoms. At the time of diagnosis, 81.9% present ptosis and 59.1% present diplopia. About 20% of the symptoms that were ocular MG at the time of diagnosis progress to generalized disease, and as a result, 85% of MG patients present generalized MG (gMG) symptoms (Practical Guideline for Myasthenia Gravis (MG) 2014 (editorial supervisor: Societas Neurologica Japonica), Nankodo Co., Ltd.; Kerty E. et al., European Journal of Neurology 2014;21:687-93). Following ocular symptoms, frequently affected muscles are the skeletal muscles of the limb. In the statistics of 2006, it is reported that 23.1 % of the patients experience weakness of neck and limb muscles at initial onset and 44.1 % at the time of diagnosis. Frequency of incidence decreases starting from difficulties in talking (dysarthria), difficulties in swallowing (dysphagia), difficulties in chewing, weakening of facial muscle, and difficulty in breathing, in this order. In the early stage of the disease, symptoms are often transient and may be ameliorated in a few weeks or more. However, symptoms are progressive and persistent, and usually peaks within a few years from disease onset in individual patients (Practical Guideline for Myasthenia gravis (MG) 2014 (editorial supervisor: Societas Neurologica Japonica), Nankodo Co., Ltd.).

In the present invention, "ocular MG" refers to MG that presents only ocular symptoms such as ptosis and diplopia, and is classified as MGFAI according to MGFA classification.

In the present invention, "generalized MG (gMG)" refers to MG that presents systemic symptoms other than ocular symptoms such as ptosis and diplopia. Ocular symptoms such as ptosis and diplopia may also be present in gMG. Ocular MG may include gMG, since there are cases where it is difficult to distinguish between ocular MG (MGFA I) and mild gMG (MGFA IIa) among gMG.

Myasthenia gravis (MG) is caused by autoantibodies against functionally important molecules present in the postsynaptic membrane of neuromuscular junctions.

Autoantibodies related to MG include autoantibodies against acetylcholine receptors (AChR), autoantibodies against muscle-specific tyrosine kinase (MuSK), autoantibodies against LDL-receptor related protein 4 (Lrp4), and the like.

While there are some differences among reports, regarding autoantibody positivity rate, about 80% of total MG are AChR-antibody positive and about 7% are MuSK-antibody positive (Gilhus N. et al., Nat Rev Dis Primers 2019;5(30)). Recently, autoantibodies against Lrp4 which forms a complex with MuSK are considered to be promising candidates as new pathogenic autoantibodies of MG, and there are reports that about 3% of total MG are Lrp4-antibody positive (Gilhus N. et al., Nat Rev Dis Primers 2019;5(30)). Further, a few percent of total MG are autoantibody negative MG cases where none of the above antibodies are detected while presenting MG symptoms.

Preexisting therapies for generalized MG are based on drug treatment according to the Practical Guideline for Myasthenia gravis (MG) 2014 (editorial supervisor: Societas Neurologica Japonica, Nankodo Co., Ltd). Therapeutic methods vary depending on expression of autoantibodies. For AChR-antibody positive generalized MG, use of anticholinesterase agents (pyridostigmine bromide, ambenonium chloride, and such) is recommended. Thereafter, treatment is performed regardless of the presence or absence of autoantibodies. Oral steroids are the first-line choice, and when symptom control is insufficient, as a second-line choice, switching to immunosuppressive agents (cyclosporin, tacrolimus hydrate, and such), and/or increasing the dose of oral steroids, and/or combining oral steroids and immunosuppressive agents is/are recommended, and in the advanced stage, steroid pulse therapy, intravenous immunoglobulin (IVIg), and blood purification therapy are recommended. Eculizumab was approved in 2017 for AChR-antibody positive gMG, and administration is carried out targeting intractable gMG. There is an international consensus guidance for treatments (Sanders DB. et al., Neurology 2016;87:419-25), and while the preference in the use of immunosuppressive agents may vary due to difference in the agents that are covered by insurance, there is no great difference in the treatment policy between Japan and the Western countries.

The pharmaceutical composition of the present invention for treatment or prevention for MG patients can be used to exert therapeutic effects such as obviate the onset of MG, or alleviate or improve clinical symptoms of patients affected with MG. Myasthenia gravis (MG) is a disease that progresses over a long time frame. The Practical Guideline for Myasthenia gravis (MG) 2014 (editorial supervisor: Societas Neurologica Japonica, Nankodo Co., Ltd) recommends treating patients "with the aim of maintaining long-term favorable QOL considering that MG is a long-lasting disease" and also recommends actively introducing relatively strong immunotherapy from the early stage of treatment. By starting treatments early, therapeutic effects such as alleviation and improvement of symptoms can be obtained and effects such as prevention or preclusion of symptom progression may be obtained as well.

Myasthenia gravis (MG) is an autoimmune disease which is difficult to achieve complete remission. Thus, even if complete remission is not achieved, alleviating or improving symptoms to a level at which minimal manifestations (MM) can be maintained, or maintaining such a state, is also included in "treatment or prevention for MG patients". For example, the Practical Guideline for Myasthenia gravis (MG) 2014 (editorial supervisor: Societas Neurologica Japonica, Nankodo Co., Ltd.) sets the goal to be achieved in MG treatment as "a level at which minimal manifestations (MM) can be maintained with 5 mg/day or less of oral prednisolone, by determining the goal of treatment considering that complete remission is difficult to achieve in MG". By achieving this treatment goal, together with improvement in social activities, clear improvement in QOL can be seen. However, even in specialized outpatient clinics in Japan, the achievement rate under current circumstances is still 40% to 50% of the entire MG cases. In order to achieve this goal, further therapeutic alternatives and therapeutic methods are desired.

Further, by administering the pharmaceutical composition of the present invention to patients that potentially have a genetic background susceptible to MG before presenting MG symptoms, the pharmaceutical composition may be used for preventing MG onset. Since MG is a disease that progresses over a long time frame, by administering the medicament of the present invention to patients that have already developed MG, progression of MG symptoms can be prevented. Further, by administering the pharmaceutical composition of the present invention to patients that have developed MG but before increase in severity (e.g., before crisis), it may be used for preventing increase in severity of MG.

More specifically, the pharmaceutical composition of the present invention for treatment or prevention for MG patients has the effect of preventing MG onset, preventing or precluding the progression of symptoms, preventing increase in severity of MG, or in therapies such as alleviation or improvement of symptoms.

Criteria for diagnosing myasthenia gravis (MG) and methods for evaluating MG are being standardized worldwide, and in the year 2000, the MG Foundation of America (MGFA) proposed the MGFA Classification for classifying MG symptoms. MGFA Classification can also be referred to as MGFA categorization, MGFA Clinical Classification, or simply as MGFA. MGFA Classification is a classification approach that classifies MG patients by the condition at the most severe state up to the present date.

As severity scores for quantitatively evaluating severity of MG, standards recommended for clinical research of MG, such as MG Activities of Daily Living (MG-ADL) scale and Quantitative Myasthenia Gravis (QMG) scores are reported (Jaretzki A. et al., Neurology 2000;55(1):16-23). MG-ADL scale is relatively easy to record as a severity score. The scores are recorded based mainly on the patient's statements, and the score of each item is added to obtain the total score. The total score can also be referred to as the MG-ADL score. The QMG score is an index that evaluates severity. The score of each item is added to obtain the total score. About 20 minutes is required to record the QMG score, and thus, it is not a brief test. Meanwhile, it has high detectability of fatigable muscles in that it can capture fatigability of extraocular muscles and muscles that seemingly have normal strength. The Myasthenia Gravis Composite (MGC) scale is used to evaluate symptoms after therapeutic intervention, which was devised by considering the advantages and disadvantages of MG-ADL scale and QMG score. The MGC scale balances well in correlation to QMG score and such, even though it allows some degree of subjective judgments by medical doctors and is thus not complicated. In addition to each of the above-mentioned scales, MG-QOL 15r and Neuro-QOL Fatigue scales will be discussed in detail in the Examples.

Efficacy of a pharmaceutical composition on MG can be evaluated using the above-mentioned evaluation items and by quantitatively measuring severity of MG before and after administration of the pharmaceutical composition to a patient and confirming whether the change in severity is statistically significant. Alternatively, the change or difference between the group administered with the pharmaceutical composition and the unadministered group (placebo) may be compared. For example, MG severity of a patient before administering the pharmaceutical composition may be determined as the baseline, and after administering the pharmaceutical composition for a certain period of time, MG severity of the patient may be quantitatively evaluated again. Alternatively, in a group of patients to be administered with the pharmaceutical composition and in a group not receiving the pharmaceutical composition, MG severity of a patient before administering the pharmaceutical composition may be determined as the baseline, and after administering the pharmaceutical composition for a certain period of time, MG severity of patients in each group may be quantitatively evaluated again. The above-mentioned evaluation criteria MG-ADL, QMG, MGC, MG-QOL 15r, and/or Neuro-QOL Fatigue may be used as standards for quantitative evaluation. In any of the above evaluation standards, when change in scores or points after administration compared to those before administration of the pharmaceutical composition (base line), or when change or difference in scores or points between groups of patients administered with or not administered with the pharmaceutical composition is statistically significant, it can be said that the pharmaceutical composition is effective against myasthenia gravis (MG). When the degree of MG of a patient is severe, MG evaluation scores or points will be high, and when the degree is mild they become low. Thus, it is desirable that the change or difference in scores or points of evaluation standards decreases.

The pharmaceutical composition of the present invention is a pharmaceutical composition for treatment or prevention for myasthenia gravis (MG) patients. Accordingly, the pharmaceutical composition of the present invention can be referred to as a pharmaceutical composition that reduces the scores or points of MG-ADL, QMG, MG-QOL 15r, Neuro-QOL Fatigue, and/or MGC in patients administered with the pharmaceutical composition of the present invention. Further, the pharmaceutical composition of the present invention can be referred to as a pharmaceutical composition that reduces the scores or points of MG-ADL, QMG, MG-QOL 15r, Neuro-QOL Fatigue, and/or MGC in patients administered with the pharmaceutical composition of the present invention, compared to the values (scores or points) measured using the same scale or scoring method obtained from the patient before administering the pharmaceutical composition of the present invention. Further, the pharmaceutical composition of the present invention can be referred to as a pharmaceutical composition that reduces the scores or points of MG-ADL, QMG, MG-QOL 15r, Neuro-QOL Fatigue, and/or MGC in patients administered with the pharmaceutical composition of the present invention compared to those of patients not administered with the pharmaceutical composition of the present invention.

The given period of administering the pharmaceutical composition for evaluating efficacy of the pharmaceutical composition on MG is not particularly limited and includes 1 week, 2 weeks, 4 weeks, 8 weeks, 12 weeks, 24 weeks, 48 weeks, 1 year, 2 years, 3 years, 4 years, and 5 years, and the period may be shorter or longer than the exemplified period.

Efficacy of the pharmaceutical composition on MG that is administered to a patient can be confirmed when the change or difference in scores or points of any of MG-ADL, QMG, MG-QOL 15r, Neuro-QOL Fatigue, and/or MGC is statistically significant. For example, the scores or points of any of MG-ADL, QMG, MG-QOL 15r, Neuro-QOL Fatigue, and/or MGC after treatment with the pharmaceutical composition may be statistically significantly reduced compared to the scores or points of the same measurement method obtained from the patient before administering the pharmaceutical composition or from a patient not administered with the pharmaceutical composition, and for example includes reduction of scores or points of 1, 2, 3, 4, 5, or 6, but may be other scores or points.

In the present invention, "as an active ingredient" means that the ingredient is contained in the pharmaceutical composition as a primal active ingredient, and the content thereof is not limited unless specifically indicated, as long as the antibodies used for the present invention are included as medicinal ingredients.

In the present invention, "standard dosing interval" refers to a dosing interval generally used for the pharmaceuticals (pharmaceutical compositions of the present invention), and includes for example, a dosing interval for constant administration that may be described in a package insert as "subcutaneous injections at four-week intervals" and such. The standard dosing interval in the present invention is not particularly limited, but examples include 1 day to 24 weeks, preferably 2 weeks to 8 weeks, more preferably 3 to 5 weeks, and even more preferably 4 weeks. The standard dosing intervals may have a certain range.

In the present invention, "dosing interval that is shorter than the standard dosing interval" refers to a dosing interval that is shorter than the dosing interval generally used (standard dosing interval) for the pharmaceuticals (pharmaceutical compositions of the present invention). The dosing interval that is shorter than the standard dosing interval is not particularly limited as long as it is shorter than the standard dosing interval, and for example, when the standard dosing interval is 24 weeks, the dosing interval that is shorter than the standard dosing interval may be shorter than 24 weeks, for example, 20 weeks. The dosing interval that is shorter than the standard dosing interval is preferably an interval that is half the standard dosing interval. For example, preferably when the standard dosing interval is 8 weeks, the dosing interval that is shorter than the standard dosing interval is 4 weeks, and more preferably when the standard dosing interval is 4 weeks, the dosing interval that is shorter than the standard dosing interval is 2 weeks. The dosing intervals that are shorter than the standard dosing intervals may have a certain range.

In the present invention, "standard dose" refers to a dose of the antibody, which is the active ingredient, generally used for the pharmaceuticals (pharmaceutical compositions of the present invention). Examples include doses of antibodies for standard administration that may be described in a package insert as "generally administered at 100 mg per dose as antibody", "generally subcutaneously injected at 100 mg per dose of antibody", and such. The standard dose in the present invention is not particularly limited, and examples include 50 to 800 mg of antibody per administration, preferably 120 to 240 mg of antibody, and more preferably 120 mg, 180 mg, or 240 mg of antibody per administration.

The "standard dose" in the present invention may vary depending on the patient's body weight. For example, the standard dose to a patient with a body weight of 100 kg or less per administration may be 120 mg of antibody and the standard dose to a patient with a body weight of more than 100 kg per administration may be 180 mg of antibody; the standard dose to a patient with a body weight of 100 kg or less per administration may be 120 mg of antibody and the standard dose to a patient with a body weight of more than 100 kg per administration may be 240 mg of antibody; and the standard dose to a patient with a body weight of 100 kg or less per administration may be 180 mg of antibody and the standard dose to a patient with a body weight of more than 100 kg per administration may be 240 mg of antibody. Further, the standard dose to a patient with a body weight of less than 100 kg per administration may be 120 mg of antibody and the standard dose to a patient with a body weight of 100 kg or more per administration may be 180 mg of antibody; the standard dose to a patient with a body weight of less than 100 kg per administration may be 120 mg of antibody and the standard dose to a patient with a body weight of 100 kg or more per administration may be 240 mg of antibody; and the standard dose to a patient with a body weight of less than 100 kg per administration may be 180 mg of antibody and the standard dose to a patient with a body weight of 100 kg or more per administration may be 240 mg of antibody. More specifically, the standard dose to a patient with a body weight of 100 kg or less (patient with a body weight of 40 kg or more and 100 kg or less) per administration may be 120 mg of antibody and the standard dose to a patient with a body weight of more than 100 kg (patient with a body weight of more than 100 kg and 160 kg or less) per administration may be 180 mg of antibody; the standard dose to a patient with a body weight of 100 kg or less (patient with a body weight of 40 kg or more and 100 kg or less) per administration may be 120 mg of antibody and the standard dose to a patient with a body weight of more than 100 kg (patient with a body weight of more than 100 kg and 160 kg or less) per administration may be 240 mg of antibody; and the standard dose to a patient with a body weight of 100 kg or less (patient with a body weight of 40 kg or more and 100 kg or less) per administration may be 180 mg of antibody and the standard dose to a patient with a body weight of more than 100 kg (patient with a body weight of more than 100 kg and 160 kg or less) per administration may be 240 mg of antibody. Further, the standard dose to a patient with a body weight of less than 100 kg (patient with a body weight of 40 kg or more and less than 100 kg) per administration may be 120 mg of antibody and the standard dose to a patient with a body weight of 100 kg or more (patient with a body weight of 100 kg or more and 160 kg or less) per administration may be 180 mg of antibody; the standard dose to a patient with a body weight of less than 100 kg (patient with a body weight of 40 kg or more and less than 100 kg) per administration may be 120 mg of antibody and the standard dose to a patient with a body weight of 100 kg or more (patient with a body weight of 100 kg or more and 160 kg or less) per administration may be 240 mg of antibody; and the standard dose to a patient with a body weight of less than 100 kg (patient with a body weight of 40 kg or more and less than 100 kg) per administration may be 180 mg of antibody and the standard dose to a patient with a body weight of 100 kg or more (patient with a body weight of 100 kg or more and 160 kg or less) per administration may be 240 mg of antibody.

The term "standard administration" in the present invention refers to an administration commonly used for the above-mentioned pharmaceuticals (pharmaceutical compositions of the present invention), for example, an administration at the above-described "standard dose" and "standard dosing interval".

In the present invention, "short-interval dosing period" refers to a period where multiple administrations are carried out prior to administering with standard dosing intervals, in which the dose is the same as the standard dose and the dosing interval is shorter than the standard dosing interval, and the period is preferably 1 to 12 weeks from the initial administration, more preferably 2 to 8 weeks, and even more preferably 4 weeks from the initial administration. A dose that is the same as the standard dose includes the case where blood concentration of the antibody is comparable to the case where standard dose is administered. The short-interval dosing period may have a certain range.

In the present invention, "(being) administered multiple times" refers to two or more administrations including the initial administration, and is preferably 2 to 5 times including the initial administration, and more preferably 3 times including the initial administration.

In the present invention, the standard administration period starts from the last administration of the short-interval dosing period. More specifically, after one standard dosing interval has passed from the last administration in the short-interval dosing period, the first administration in the standard administration period is carried out. For example, in the case of performing 3 administrations including the initial dose in a short-interval dosing period, after the 3rd administration, one standard dosing interval lapses, and the period at which administration is performed at standard dosing intervals from the 4th administration and onward will become the standard administration period. For example, in the case where the short-interval dosing period is 4 weeks, administration including the initial administration in the short-interval dosing period is 3 times, the standard dosing intervals is 4 weeks, and the dosing interval that is shorter than the standard dosing intervals is 2 weeks, the initial, 2nd, and 3rd administrations are performed with 2 weeks intervals within the 4 weeks which is the short-interval dosing period, and then the 4th administration is performed after 4 weeks has passed from the 3rd administration, which is the 1st standard dosing interval, and thereafter, administration will be performed with the standard dosing intervals of 4 weeks. Accordingly, after the 3rd administration which is the last administration of the short-interval dosing period, the period at which administration is carried out with 4 weeks intervals, which is the standard dosing interval, will become the standard administration period.

The pharmaceutical composition of the present invention is preferably a pharmaceutical composition the administration of which is carried out by administering an antibody for 2 to 5 times in a short-interval dosing period from the initial administration with an interval of 1 to 3 weeks at the same dose as a standard dose, and then from the last administration of the short-interval dosing period, performing standard administration with an interval of 2 to 8 weeks of the antibody at 50 to 800 mg per administration, which is the standard dose. More preferably, the pharmaceutical composition is such that its administration includes administering SA237 for 3 times in a short-interval dosing period from the initial administration with an interval of 2 weeks (i.e., administration at week 0, week 2, and week 4) at the same dose as a standard dose, and then from the last administration of the short-interval dosing period, performing standard administration with an interval of 4 weeks (i.e., continued with a 4 weeks interval counting from the initial administration of the short-interval period at week 8, week 12, week 16) of SA237 at 120 mg, 180 mg, or 240 mg per administration, which is the standard dose.

The preferred administration schedule for the antibody of the present invention can be adjusted, for example, by appropriately extending the administration interval by monitoring the conditions of the disease and changes in the blood test values.

The present invention also provides an article of manufacture (such as a kit, a device, and the like) for use in a method of the present invention, which contains a pharmaceutical composition or a medicament of the present invention. The pharmaceutical composition or medicament of the present invention comprises an IL-6 inhibitor as described herein. The article of manufacture may be packaged with an additional pharmaceutically acceptable carrier or medium, or an instruction manual describing how to use the kit, the device, or the like, etc.

In one embodiment, the article of manufacture comprises a container and a label on or a package insert accompanying the container. Suitable containers include, for example, bottles, vials, syringes (including a prefilled syringe and an autoinjector), IV solution bags, etc. The containers may be formed from a variety of materials such as glass or plastic. In one embodiment, the container may hold a composition alone or in combination with another composition effective for treating, preventing and/or diagnosing the condition, and may also have a sterile access port (for example, the container may be a syringe, an autoinjector, an intravenous solution bag or a vial having a stopper pierceable by a hypodermic injection needle). At least one active ingredient in the composition is an IL-6 inhibitor as described in the present disclosure, preferably an anti-IL-6 receptor antibody, and more preferably satralizumab.

In one embodiment, a device as the article of manufacture of the present invention as described above may be a prefilled syringe for injection via any administration route (such as intravenous, subcutaneous, or the like) which comprises a fixed dose of an IL-6 inhibitor as described in the present disclosure, preferably an anti-IL-6 receptor antibody, and more preferably satralizumab, in a pharmaceutically acceptable excipient. In another embodiment, the device may be an autoinjector for subcutaneous administration which comprises a fixed dose of an IL-6 inhibitor as described in the present disclosure, preferably an anti-IL-6 receptor antibody, and more preferably satralizumab, in a pharmaceutically acceptable excipient. In certain embodiments, the device (such as a prefilled syringe and autoinjector) may comprise 120 mg, 180 mg, or 240 mg of satralizumab.

In one embodiment, the label or package insert indicates that the pharmaceutical composition or medicament is used for treating the condition of choice. Moreover, the article of manufacture may comprise (a) a first container with a composition contained therein, wherein the composition comprises an IL-6 inhibitor as described above, preferably an anti-IL-6 receptor antibody, and more preferably satralizumab; and (b) a second container with a composition contained therein, wherein the composition comprises a further therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The term "package insert" is used to refer to instructions customarily included in commercial packages of therapeutic products, that contain information about the indications, usage, dosage, administration, combination therapy, contraindications and/or warnings concerning the use of such therapeutic products.

Pharmaceutical compositions of the present invention used for therapeutic or preventive purposes can be formulated to produce freeze-dried formulations or solution formulations by mixing, if necessary, with suitable pharmaceutically acceptable carriers, vehicles, and such. The suitable pharmaceutically acceptable carriers and vehicles include, for example, sterilized water, physiological saline, stabilizers, excipients, antioxidants (such as ascorbic acid), buffers (such as phosphate, citrate, histidine, and other organic acids), antiseptics, surfactants (such as PEG and Tween), chelating agents (such as EDTA), and binders. Other low-molecular-weight polypeptides, proteins such as serum albumin, gelatin, and immunoglobulins, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol may also be contained. When preparing an aqueous solution for injection, physiological saline and isotonic solutions comprising glucose and other adjuvants such as D-sorbitol, D-mannose, D-mannitol, and sodium chloride may be used; and appropriate solubilizers such as alcohol (for example, ethanol), polyalcohols (such as propylene glycol and PEG), and nonionic surfactants (such as polysorbate 80, polysorbate 20, poloxamer 188, and HCO-50) may be used in combination. By mixing hyaluronidase into the formulation, a larger fluid volume can be administered subcutaneously (Expert Opin. Drug Deliv. 2007 Jul; 4(4): 427-40). Furthermore, syringes may be prefilled with the pharmaceutical composition of the present invention. Solution formulations can be prepared according to the method described in WO2011/090088.

If necessary, the pharmaceutical compositions of the present invention may be encapsulated in microcapsules (e.g., those made of hydroxymethylcellulose, gelatin, and poly(methylmethacrylate)), or incorporated into colloidal drug delivery systems (e.g., liposomes, albumin microspheres, microemulsions, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also known, and such methods may be applied to the pharmaceutical compositions of the present invention (Langer et al., J. Biomed. Mater. Res. 15: 267-277 (1981); Langer, Chemtech. 12: 98-105 (1982); U.S. Patent No. 3,773,919; European Patent Application Publication No. EP 58,481; Sidman et al., Biopolymers 22: 547-556 (1983); and EP 133,988).

The pharmaceutical composition of the present invention can be administered to a patient *via* any appropriate route. For example, it can be administered to a patient intravenously by bolus injection or by continuous infusion, intramuscularly, intraperitoneally, intracerebrospinally, transdermally, subcutaneously, intraarticularly, sublingually, intrasynovially, orally, by inhalation, locally, or externally, for a certain period of time. Intravenous administration or subcutaneous administration is preferred.

The antibody of the present invention may be used in an agent comprising the antibody for treating or preventing MG, or may be used in a method of treating or preventing MG which comprises administering the antibody to a patient with MG. In addition, the antibody of the present invention may be used in the manufacture of an agent for treating or preventing MG. Further, the antibody of the present invention may be referred to as an antibody for use in the treatment of a patient with MG or for use in the prevention of MG. Further, the antibody of the present invention is preferably satralizumab, and is specifically an antibody comprising a heavy chain variable region comprising the amino acid sequence of SEQ ID NO: 1 and a light chain variable region comprising the amino acid sequence of SEQ ID NO: 2.

All prior art references cited herein are incorporated by reference into the present specification.

### [Example]

Herein below, the present invention will be specifically described with reference to the Examples, but it is not to be construed as being limited thereto.

### Example 1: Preparation of satralizumab (SA237)

SA237, which is an IL-6 receptor antibody described in the patent document WO 2010/035769 (an antibody comprising a heavy chain having the amino acid sequence of SEQ ID NO: 26 (SEQ ID NO: 3 in the present specification) and a light chain having the amino acid sequence of SEQ ID NO: 29 (SEQ ID NO: 4 in the present specification) in the patent document WO 2010/035769), was prepared according to the description of the patent document. The amino acid sequence of the heavy chain variable region is shown in SEQ ID NO: 1, and the amino acid sequence of the light chain variable region is shown in SEQ ID NO: 2. Using the prepared antibody, a subcutaneous administration preparation was prepared by the method described in the patent document WO 2011/090088.

### Example 2: Phase III, randomized, double-blinded, placebo-controlled, multicenter study Study Design

### 1. Description of the Study

The Phase III, randomized, double-blinded, placebo-controlled, multicenter study is designed to evaluate the efficacy, safety, pharmacokinetics, and pharmacodynamics of satralizumab compared with placebo as add-on therapy to standard of care (SOC) for the treatment of generalized myasthenia gravis (generalized MG or gMG). The study includes a 24-week double blind (DB) period, and an open-label extension (OLE) period.

A summary of the study design is illustrated in Figure 1.

### 1.1 Double Blind Period

During the double blind treatment period, patients are randomized in a 1:1 ratio to receive 120 mg, 180 mg, or 240 mg satralizumab based on the body weight (Group A), or placebo (Group B) for 24 weeks. Randomization will be stratified based on the baseline standard-of-care therapy (SOC), auto-antibody type, and the region as the stratification factors.

A blinded drug is administered subcutaneously to patients at Weeks 0, 2, 4, and every four weeks thereafter until the end of the DB period, in addition to SOC at a stable dose. During the DB period, an interim analysis of pharmacokinetic (PK) data is performed. Based on the results from the interim PK analysis and pre-specified criteria, whether the study drug dose is increased is determined. If it is determined to be increased, thereafter, Group A is administered with either 180 mg or 240 mg satralizumab.

The background therapies permitted in this study are anticholinesterase inhibitors (AChEI) alone or the following treatment methods (with or without AChEI): oral corticosteroids (OCSs), one immunosuppressant therapy (IST), and an OCS in combination with one IST. The permitted IST drugs are, for example, azathioprine, mycophenolate mofetil, cyclosporin A, and tacrolimus, but are not limited thereto.

### 1.2 Open-Label Extension Period

Patients who completed the DB period can enter the OLE period. Upon completion of the Week 24 assessments, all patients receive open-label treatment with satralizumab.

In the OLE period, all patients receive open-label treatment with 120 mg, 180 mg, or 240 mg satralizumab.

Based on the investigator's judgment, dose reduction (taper) of background therapy (OCS, IST, and/or AChEI) can be started at Week 12 or later in the OLE period.

### 1.3 Unscheduled Visits and Rescue Therapy

Unscheduled visits can be performed at the discretion of the treating neurologist. If a suspected gMG exacerbation occurs during the study, the participant needs to return to the study site and undergo an evaluation of gMG severity before or immediately after receiving rescue therapy.

The rescue therapy includes intravenous immunoglobulin therapy (IVIg), and plasma exchange (PE) therapy with or without high-dose corticosteroids. The choice of rescue therapy is determined by the investigator on the basis of the overall clinical assessment.

Patients who received rescue therapy during the DB period may receive OLE satralizumab administration after completion of the DB period.

### 1.4 Subject Patients and Study Regions

The study is conducted in countries including, but not limited to, North America, Europe, Latin America, and Asia.

This study enrolls approximately 240 patients, including approximately 20 adolescents aged 12 to 17 years with gMG. The main selection/exclusion criteria are shown below.

### [Selection criteria]

- Patients aged 12 years or older at the time of consent acquisition
- Patients diagnosed with myasthenia gravis with generalized muscle weakness that falls under Class II, III, or IV severity classification by the Myasthenia Gravis Foundation of America (MGFA). Diagnostic confirmation needs to be demonstrated and supported by a positive serum test for one of the three antibody types, AChR antibody, MuSK antibody, and Lrp4 antibody at the time of screening.
- Patients with a total MG-ADL score of 5 or higher, and no less than half of the scores associated with non-ocular symptoms.
- Patients that can receive the rescue therapies (immunoglobulin therapy (IVIg), plasma exchange (PE) therapy, and high-dose corticosteroid therapy).
- Patients having received, prior to enrollment, stable doses of treatment for severe myasthenia gravis (azathiopurine, mofetil mycophenolate, cyclosporin A, tachlorimus, oral corticosteroids (OCS), or anticholinesterase inhibitors (AChEI)).

### [Exclusion criteria]

- Patients with a history of thymic cyst, thymoma, or other thymic neoplasm (as defined by the WHO classification for thymic tumor (2015)) are excluded, except where they are considered to have been cured by appropriate treatment, and there is no observation of recurrence for five years or more prior to screening.
- Patients after less than twelve months of thymectomy are excluded.
- Patients with a history of myasthenia crisis (MGFA class V) within the last three months are excluded.

### 2. Length of Study

The total length of study, from the first patient screening to the end of the OLE period, is estimated to be approximately four years.

### 3 Rationale for Study Design

### 3.1 Rationale for Satralizumab Dose and Schedule

Weight-tiered dosing via SC injection is used in this study for the investigation of efficacy and safety of satralizumab in gMG as shown in Table 1.

**[Table 1]**

| Dosing Regimen in Phase III Study of Satralizumab for the Treatment of generalized Myasthenia Gravis | |
|---|---|
| Body weight at Baseline^{a} | Dose and Regimen |
| 100 kg or less | 120 mg administered at Weeks 0, 2, 4, and Q4W thereafter by subcutaneous injection |
| More than 100 kg | 180 mg administered at Weeks 0, 2, 4, and Q4W thereafter by subcutaneous injection |

| | |
|---|---|
| Q4W: Every four weeks. a: In case of change in the body weight of an individual patient, recommendation is made to include him/her in the other dosing group. See Table 2. | |

The dosing regimen is based on a combination of sources of information including the following:
PK, PD, and safety data for satralizumab for the initial development in neuromyelitis optica spectrum disorder (NMOSD); and
Consideration of differences in population demographics.

The 120-mg fixed dose regimen investigated in the Phase III studies in NMOSD was associated with high predicted median trough receptor occupancy (95% or more) at steady-state (RO_{tr,ss}) values in most patients, and was shown to be safe and efficacious in all body weight groups. The few patients with predicted RO_{tr,ss} values of less than 80% generally had baseline body weights of more than 100 kg.

Real-world data from a large U.S. emergency room-based dataset of 58,860 patients having an MG code suggest that approximately 30% of the patients in this population may have body weights of more than 100 kg. Exposure similar to that in NMOSD is expected to be effective also in gMG, and therefore simulations were performed using the existing population-PK (pop-PK) model to estimate the dose required for achieving the same maximal RO_{tr,ss} values achieved in NMOSD patients throughout the dose interval for gMG patients across the expected body weight range.

The predicted maximum concentration observed (Cₘₐₓ), trough concentration (C_{trough}), and RO values in serum following administration of 120 mg every four weeks and 180 mg every four weeks in patients weighing 100 kg or less and more than 100 kg, respectively, are shown in Figure 2. It was suggested that similar RO can be achieved by administering 180 mg to the patients weighing more than 100 kg compared to the administration of 120 mg to the patients weighing 100 kg or less. Furthermore, the exposures in administering 180 mg to the patients weighing more than 100 kg were within the range of exposures by 120 mg administration that has been confirmed to be safe in the Phase III studies in NMOSD patients. The study design includes an PK interim analysis to confirm whether the target exposures and RO are achieved.

A favorable safety profile was demonstrated in the NMOSD program for satralizumab, supporting the targeting of similar exposures in this study. However, exposures below the target range may warrant an increase in dose to optimize IL-6 blockade throughout the dose interval across the expected body weight range. Therefore, an option for dose increase is included (for all body weight ranges, if necessary) in the study design. Details are provided in Section 3.11.

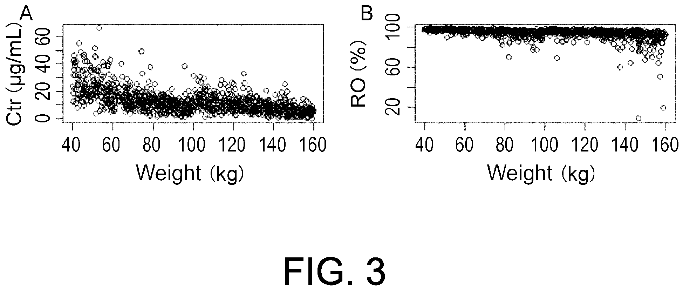

While most PK parameter estimates for satralizumab were shown to be very similar between healthy adults (HV) and patients with NMOSD, population differences in the total clearance of drug (CL) were observed (covariate value [95%CI] in HV 95.8%[67.5 to 124.1]). Therefore, PK simulation has been used to explore potentially useful regimens, presuming the case where CL in the gMG population was similar to CL in HV. The simulations presented in Figure 3 indicate that, if that is the case, a dosing regimen of 180 mg and 240 mg (180/240 mg regimen) for patients weighing 100 kg or less and more than 100 kg, respectively, would be expected to maintain the target level of RO across the body weight ranges. The dose is adapted to this higher dosing regimen if CL in gMG is reflective of CL in HV.

PK simulation is used as part of the PK interim analysis to confirm whether the initially proposed doses achieved target exposures, or the dose should be adapted to 180 mg or 240 mg regimen. In either case, the chosen dosing regimen is expected not to significantly exceed the exposure confirmed to be safe in the Phase III studies in NMOSD patients.

### 3.2 Rationale for Choice of Background Treatment

Patients on a stable dose of background therapy are enrolled. The background SOC therapies permitted in the study are the following:
AChEI
OCS
one IST
OCS in combination with one IST

Concomitant use of AChEI is permitted for patients on a stable dose with OCS, one IST, and OCS in combination with one IST.

The combination treatment regimen of IST and/or OCS was chosen on the basis of being the most common treatment choice for gMG worldwide and in accordance with the international consensus guidance for management of MG (Sanders et al., Neurology 2016; 87: 419-25).

### 3.3 Rationale for Primary Outcome Measure: Myasthenia Gravis Activities of Daily Living (MG-ADL)

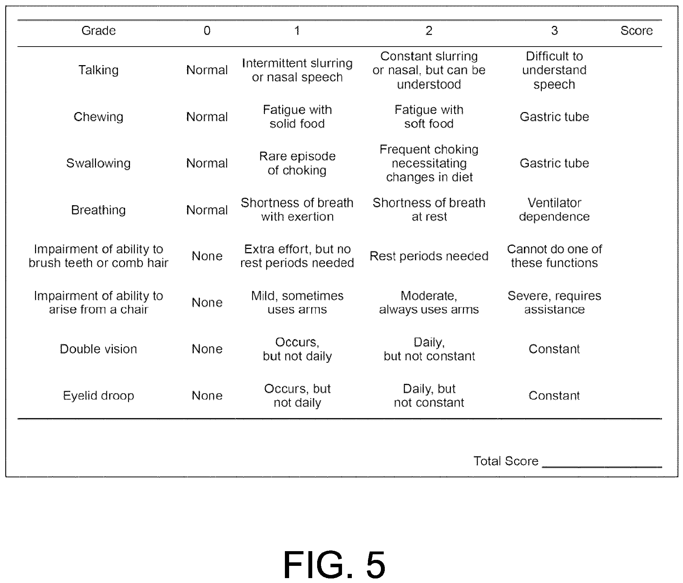

The primary objective for this study is to compare the efficacy of satralizumab added to SOC versus placebo added to SOC using the MG-ADL as a primary outcome measure. A sample of MG-ADL questionnaire is shown in Figure 5.

The MG-ADL was developed by Wolfe and colleagues (Neurology 1999; 52: 1487-9) to assess the degree of gMG symptoms (six items: diplopia, ptosis, difficulties with chewing, swallowing, talking, and respiratory problems) and functional limitations in carrying out activities of daily living (two items: disability to brush teeth or comb hair and impairment in the ability to arise from a chair) that have been shown to be present and clinically relevant in gMG patients. Each of the eight items is ranked on a 0 to 3 scale yielding a total score that ranges from 0 to 24, and higher scores indicate greater disease severity (Figure 5). The items of the MG-ADL were all derived from the original 13-item symptom list that comprises the clinician-rated QMG scale.

The psychometric properties of the MG-ADL have been characterized. Construct validity has been demonstrated by showing correlations with the QMG (r=0.58; Wolfe et al., Neurology 1999; 52: 1487-9), MGC measure (r=0.85), and the MG-QOL 15r measure (r=0.76) (Muppidi et al., Muscle Nerve 2011; 44: 727-31). Test/retest reliability was demonstrated in a small sample (n=26) of patients with two repeated assessments separated by 2 to 4 days, showing a high degree of reproducibility (r=0.94) (Muppidi et al., Muscle Nerve 2011; 44: 727-31).

Because of the importance of patients' subjective feedback due to the fluctuating nature of the disease and the established psychometric properties including good content validity associated with patients' functioning in daily life, MG-ADL is an appropriate primary outcome measure. MG-ADL scale has been used as the primary outcome measure in the recently completed (Howard et al., Lancet Neurol 2017; 16: 976-86) and in the ongoing Phase III gMG randomized, placebo-controlled trials (NCT03669588, NCT03971422, NCT03920293, NCT04115293, and NCT03304054).

### 3.4 Rationale for Secondary Outcome Measures

The QMG, MG-QOL 15r, NeuroQOL Fatigue scale, and the MGC were selected as secondary endpoints in order to compare the efficacy of satralizumab added to SOC versus placebo added to SOC.

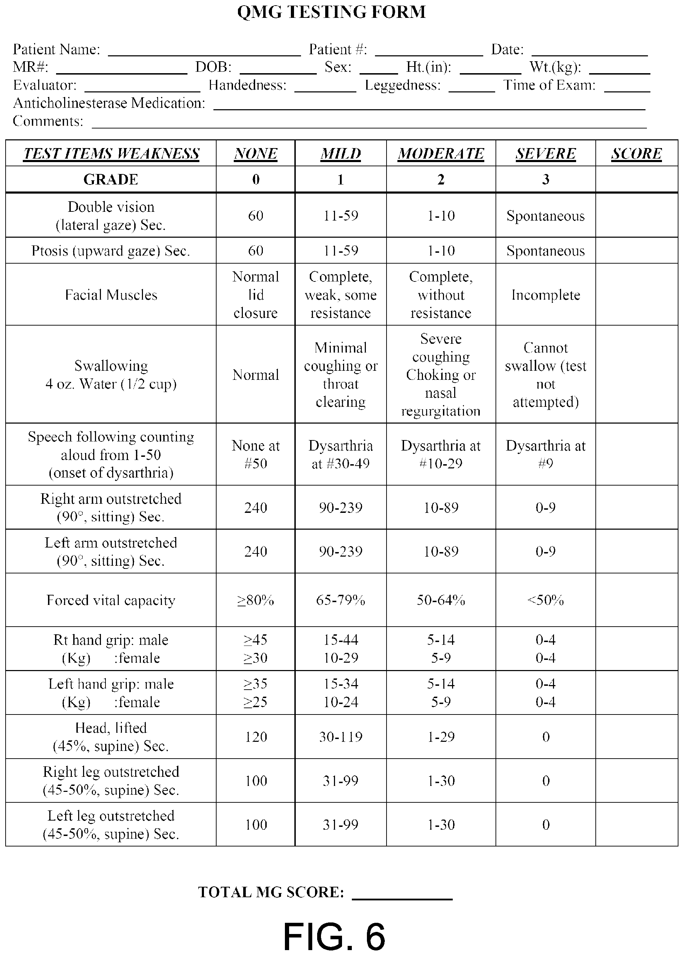

The QMG is a 13-item assessment of gMG symptom severity based on clinical examination (Tindall et al., N Engl J Med 1987; 316: 719-24). It has been used in clinical trials since 1983, when it was first developed to study the relationship between AChR-Ab binding and disease severity (Besinger et al., Neurology 1983; 33: 1316-21). Recently, the QMG has been used as a key secondary outcome measure in the recently completed study of eculizumab for gMG (Howard et al., Lancet Neurol 2017; 16: 976-86) and all ongoing Phase III gMG randomized, placebo-controlled trials (NCT03669588, NCT03971422, NCT03920293, NCT04115293, and NCT03304054). A sample of QMB questionnaire is shown in Figure 6.

The QMG assesses severity of symptoms ranging from 0 to 3 for ptosis, diplopia, orbicularis oculi weakness, swallowing, speech disruption, percent forced vital capacity, arm and leg endurance (four items), grip strength (two items), and neck flexion strength, resulting in a total score that ranges from 0 to 39, and higher values indicate severer symptoms (Tindall et al., N Engl J Med 1987; 316: 719-24).

The psychometric properties of the QMG have been studied using both observational and clinical trial data. The QMG has acceptable internal consistency (Cronbach's α=0.74) and test/retest reliability (intra-class correlation coefficient [ICC] = 0.88) in clinically stable patients (Barnett et al., J Neuromuscular Disease 2015; 2: 301-11). Construct validity studies of the QMG found correlations with the MGFA score (r²=0.54) and the MG-QOL 15 (r=0.41) (Barnett et al., J Clin Neuromuscular Dis. 2012; 13: 201-5).

This study evaluates the efficacy of satralizumab added to SOC versus placebo added to SOC by comparing the change from baseline in the QMG score at Week 24 (the end of the DB period).

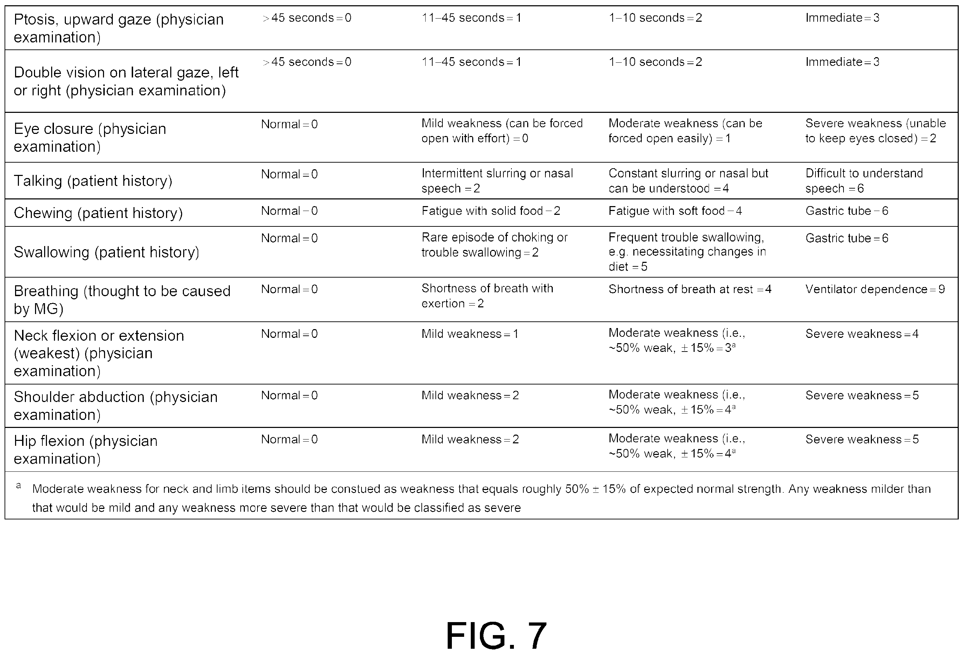

The MGC is an additional secondary efficacy outcome measure in this trial. As the name implies, the MGC is a composite measure consisting of items drawn from the MG-ADL (chewing, swallowing, speech, and breathing), QMG (diplopia and ptosis), and Manual Muscle Test (hip, neck, facial, and deltoid strength) to include both clinician- and patient-reported elements in a single measure (Burns et al., Muscle and Nerve 2008; 38: 957-63). The ten items compose a total score ranging from 0 to 50, and higher values indicate increased symptom severity. A sample of MGC questionnaire is shown in Figure 7. The psychometric properties of the MGC were evaluated in a primary care setting in 175 patients spread across 11 sites (Burns et al., Neurology 2010; 74: 1434-40). Test/retest reliability was found to be high (0.98 correlation coefficient) in a small sample of patients from a single site (n=38). The MGC exhibited moderate-to-strong convergent validity with the MG-ADL total score (r=0.85), MG-QOL 15 total score (r=0.68), and the Manual Muscle Test (r=0.8).

Since the MGC has fair psychometric properties and is drawn from both physician- and patient-reported items, the Medical Scientific Advisory Board of the Myasthenia Gravis Foundation of America has recommended the use of MGC in randomized clinical trials of potential new gMG therapies (Benatar et al. 2012). As one of the secondary efficacy objectives, this study evaluates the efficacy of satralizumab added to SOC versus placebo added to SOC by comparing the change from baseline in the MGC score at Week 24 (the end of the DB study period).

The MG-QOL 15 is a disease-specific health-related QOL measure that consists of 15 items: mobility (9 items), symptoms (3 items), and contentment and emotional well-being (3 items). Items are scored on a Likert scale from 0 to 4 with the total score ranging from 0 to 60, and higher total scores indicate lower HRQoL (Burns et al., 2008). The MG-QOL 15 has strong internal consistency reliability (Cronbach's α=0.89) and test/retest reliability (ICC=0.98) (Burns et al., 2011). In a recent clinical trial of mycophenolate mofetil in gMG, the MG-QOL 15 correlated well with the physical and mental summary components of the 36-Item Short Form Survey, and with MG-specific measures (QMG, MG-ADL) (Burns et al., Muscle and Nerve 2008; 38: 957-63).

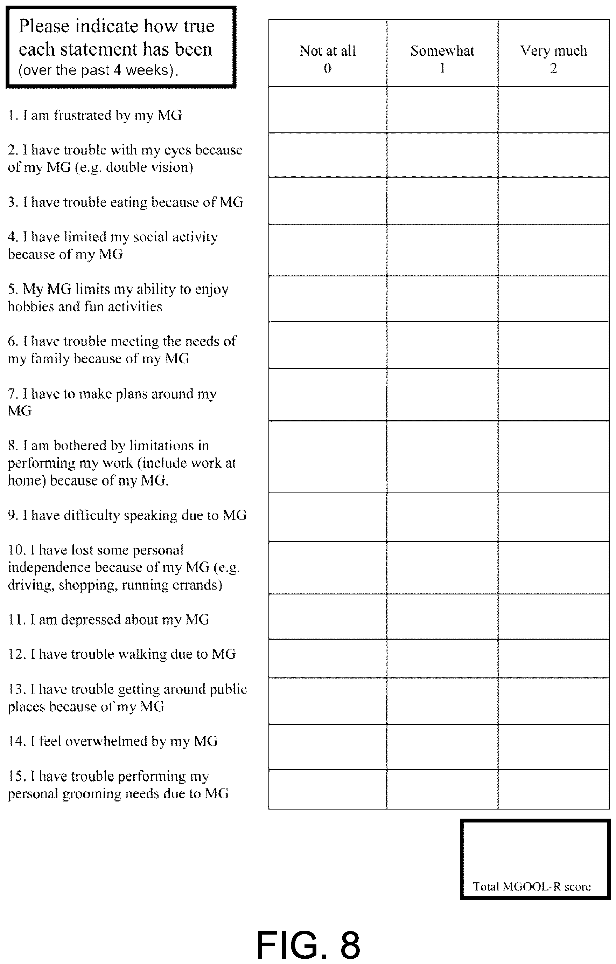

The MG-QOL 15 also demonstrated to be reliable in a randomized, controlled study of IVIg versus PE. In the study, responders to treatment improved on average by nine points compared with non-responders who changed by two points, suggesting that a decrease in MG-QOL 15 by seven or more points is correlated with improvement in the subgroup with moderate-to-severe MG (QMG score of less than 11) (Barnett et al., 2013). The minimal important difference has not been fully determined. Due to its extensive use, the MG-QOL has been recently modified to improve the performance of certain items (Burns et al., 2016). In the modified version (MG-QOL 15r), the score ranges of the 15 items were altered from 0-4 to 0-2 based on Rasch analysis. The resulting measure scores range from 0 to 30, and higher scores indicate lower health-related quality of life. When compared with the original scale, the modified version has better psychometric properties than the original and it is very easy to use (Burns et al., 2016). A sample of MG-QOL 15r questionnaire is shown in Figure 8.

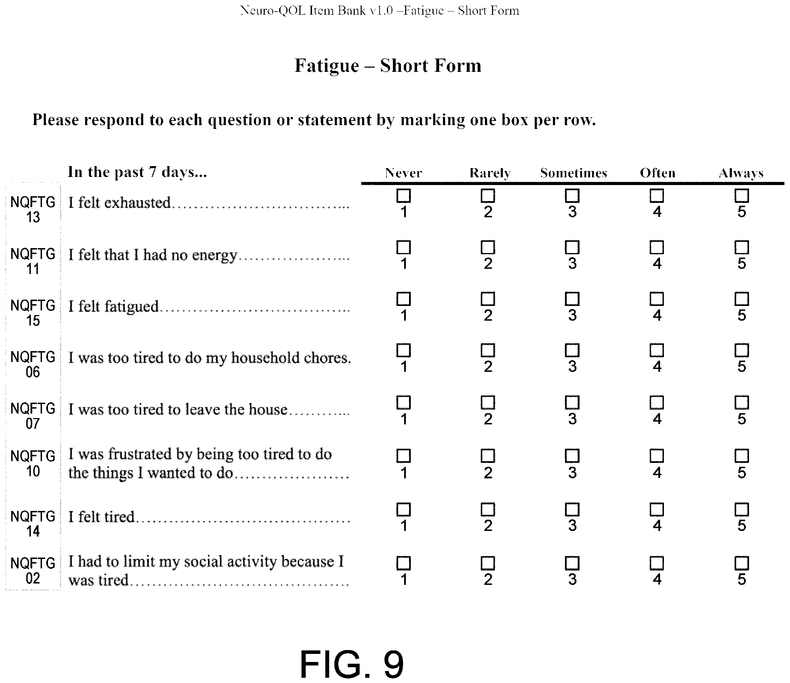

The Neuro-QOL Fatigue scale is a short form that is part of a collection of instruments and item banks that was developed through a National Institute of Neurological Disorders and Stroke-sponsored initiative to evaluate the HRQoL of adults and children diagnosed with neurological disorders (Cella et al., Neurology 2012; 78: 1860-7). It consists of eight items, each using a 5-level Likert scale ranging from 1 (never) to 5 (always), with a 7-day recall period. The measure has demonstrated strong internal consistency reliability (Chronbach's α coefficient=0.97) and construct validity (see, e.g., Cella et al., Neurology 2012; 78: 1860-7). A sample of the Neuro-QOL Fatigue scale is shown in Figure 9.

The scale has recently been assessed in patients with MG using an observational cohort of 257 patients receiving either IVIg/PE or prednisone (Tran et al., Muscle Nerve 2018; 58: 197-203). The results demonstrated a positive relationship between fatigue and MG severity. Patients in MGFA Classes II and III exhibited mild-to-moderate fatigue, while those in Class IV experienced severe fatigue. Fatigue scores were shown to correlate positively with MG Impairment Index, QMG, MGC, and MG-ADL (Pearson's r=0.52-0.69), indicating acceptable convergent validity (Tran et al., Muscle Nerve 2018; 58: 197-203). All treatment groups showed a significant decrease in fatigue at Week 4 compared to baseline, a standardized response mean (SRM) for the overall population was 0.49, suggesting that the measure has good responsiveness (Tran et al., 2018, Muscle Nerve 2018; 58: 197-203).

The Neuro-QOL Fatigue scale was also used in the recent REGAIN study of eculizumab in MG patients (Andersen et al., Qual Life Res. 2019; 28: 2247-54).

### 3.5 Secondary Responder Analysis

As part of the secondary efficacy objectives, supportive responder analyses for the MG-ADL, QMG, and MGC total scores are performed.
They include the following, but are not limited thereto:
Variation from baseline of total MG-ADL score, and proportion of patients with a decrease by certain points or more from the baseline, in the overall population or population with no rescue therapy received
Variation from baseline of total QMG score, and proportion of patients with a decrease by certain points or more from the baseline, in the overall population or population with no rescue therapy received
Variation from baseline of total MGC score, and proportion of patients with a decrease by certain points or more from the baseline, in the overall population or population with no rescue therapy received
Variation from baseline of total MG-QOL 15r score, and proportion of patients with a decrease by certain points or more from the baseline, in the overall population or population with no rescue therapy received
Variation from baseline of Neuro-QOL Fatigue Subscale total score, and proportion of patients with a decrease by certain points or more from the baseline, in the overall population or population with no rescue therapy received

### 3.6 Rationale for Open-Label Extension

Following the 24-week DB period, the study includes an OLE period of approximately 2 years from the last patient in the global study. An objective of the OLE is to evaluate satralizumab long-term safety and efficacy, including steroid/IST reduction, in gMG patients.

This study enrolls patients receiving stable background therapy, including OCS and IST indicated in "1.1 Double-Blind Period".

Long-term steroid treatment is associated with numerous adverse effects on many organ systems, including bone (osteoporosis, avascular necrosis), muscle (myopathy), metabolism and endocrine organs (weight gain, impaired glucose tolerance, hypothalamic-pituitary-adrenal suppression), skin (skin atrophy, acne, striae), eyes (glaucoma, cataract), behavior and mood (mood disorders, insomnia), cardiovascular system (hypertension, fluid retention, perturbations of serum lipoproteins), gastrointestinal system (gastritis, peptic ulcer disease), and immune system (increased risk of infection). One of the key treatment goals in MG is to reduce exposure to steroids (Sanders et al., Neurology 2016; 87: 419-25; Jaretzki et al., 2000 Neurology 2000; 55: 16-23). The adverse event profile of the steroid-sparing IST that constitutes the background therapy in this study includes renal toxicity with the use of cyclosporine, myelosuppression, infections, liver toxicity, and malignancies (Vodopivec et al., 2014 Semin Nerol 2014; 34: 467-78; Collins et al., 2019 Dermatol Clin 2019; 37: 83-94).

At or after Week 12 of the OLE, tapering of steroids and ISTs background treatment is allowed based on clinical judgement.

The ability of patients to successfully taper steroids or ISTs while concurrently taking satralizumab is evaluated in the OLE period based on analyses that are pre-specified in the Statistical Analysis Plan (SAP).

### 3.7 Rationale for Biomarker Assessments

This study assesses whether biomarkers measured at baseline can be used to identify patients who can receive clinical benefit when treated with satralizumab, or identify differential disease progression. The study also assesses whether biomarkers can aid in characterizing the mechanism of action of satralizumab in gMG, provide evidence of satralizumab activity in gMG, or increase the knowledge and understanding of gMG disease biology.

Exploratory biomarker samples are used for research purposes to identify pathway and/or disease biomarkers (including, but not be limited to, biomarkers reflective of inflammation, B-cell and T-cell subsets, activities, and products (e.g., serum IL-17 and B-cell subsets in blood)).

PD biomarker samples are collected for the assessment of target binding (e.g., IL-6 and sIL-6R) in response to satralizumab treatment.

### 3.8 Rationale for PK Sample Collection Schedule

Samples for assessing serum satralizumab concentration are obtained prior to each administration during the DB period and up to Week 24 of the OLE period, and every 12 weeks for the remaining duration of treatment, to explore the pharmacokinetics of satralizumab in the gMG population following longer term treatment. This assessment includes the impact of a range of covariates on exposure (e.g., gender, race, age, and body weight), and relationship between exposure and PD, efficacy, immunogenicity, and safety endpoints.

### 3.9 Rationale for PK Interim analysis

The dosage and administration of the Phase III studies conducted on NMOSD patients were determined from the Phase I study, and a good efficacy/safety profile was shown in the Phase III studies. While a similar dose selection approach is proposed for this Phase III study in gMG, the sponsor is mindful that population differences in the pharmacokinetics for satralizumab exist, and therefore, the proposed Phase III study design in gMG includes performing a PK interim analysis. The PK interim analysis is performed to ensure that patients are achieving target exposures while the sponsor remains blinded, maintaining the integrity of this pivotal study.

An interim analysis of PK data is performed when approximately 60 patients have completed at least 8 weeks of DB treatment (including approximately 30 patients from the satralizumab group). The purpose of the interim analysis is to assess whether the achieved exposure to satralizumab (and predicted RO) is within the predicted range. The use of the existing RO model for prediction of RO in this interim analysis is considered appropriate since the target is the same in both indications, and similar target expression is expected for gMG.

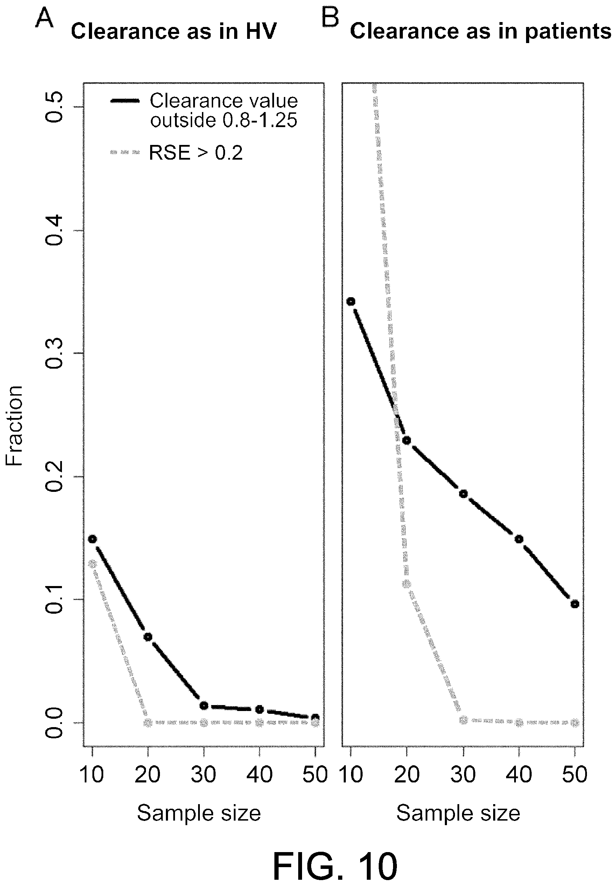

The number of cases proposed for this PK interim analysis is supported by the PK simulation shown in Figure 10. Assuming that the CL in gMG patients is similar to CL observed in either healthy adults or NMOSD patients, it was considered that a PK interim analysis on approximately 30 patients can detect an increase in CL.

Throughout the study, and therefore for the PK interim analysis, patients of a range of bodyweights in proportions approximately representative of the overall gMG population are enrolled.

The procedure of decision of dosage and administration is defined in a statistical analysis plan prior to study start. PK data obtained from gMG patients are incorporated into an existing population pharmacokinetic model to update the model, and the obtained predicted exposure and RO are compared with the preset criteria to determine the optimal dose. If target exposures are not achieved, the dose is increased to the pre-defined dose regimen of 180 mg and 240 mg for patients weighing 100 kg or less and more than 100 kg, respectively. The rationale for the dosage and administration in the case that CL values in gMG are reflective of those in HV (which are higher than those in the NMOSD population) is described in Section 3.1.

### 3.10 Rationale for Immunogenicity Sample Collection

Anti-satralizumab antibodies (ADAs) were detected in a large proportion of the NMOSD patients enrolled in the Phase III studies, and data indicating that the probability of developing ADAs was positively correlated with low exposure and higher body weight were obtained. However, similar clinically important efficacy was demonstrated in all body weight groups in these Phase III studies.

Serum samples for ADAs are taken in parallel to PK samples to assess the incidence and titer-time profile of ADAs in the gMG population, and the impact on exposure to satralizumab. ADA data are included in the blinded review of PK data at Week 8 for the purpose of interpretation of the satralizumab concentration data, and also included in the subsequent analysis based on the full study data set.

### [Industrial Applicability]

The present invention can provide pharmaceutical compositions for treating or preventing myasthenia gravis with a mechanism of action different from that of existing therapeutic drugs.

## Claims

1. A pharmaceutical composition for treatment or prevention for a patient with myasthenia gravis comprising, as an active ingredient:
(i) an antibody comprising heavy chain CDR1 comprising the amino acid sequence of SEQ ID NO: 5, heavy chain CDR2 comprising the amino acid sequence of SEQ ID NO: 6, heavy chain CDR3 comprising the amino acid sequence of SEQ ID NO: 7, light chain CDR1 comprising the amino acid sequence of SEQ ID NO: 8, light chain CDR2 comprising the amino acid sequence of SEQ ID NO: 9, and light chain CDR3 comprising the amino acid sequence of SEQ ID NO: 10;
(ii) an antibody comprising a heavy chain variable region comprising the amino acid sequence of SEQ ID NO: 1 and a light chain variable region comprising the amino acid sequence of SEQ ID NO: 2; or
(iii) an antibody comprising a heavy chain comprising the amino acid sequence of SEQ ID NO: 3 and a light chain comprising the amino acid sequence of SEQ ID NO: 4,
wherein the patient is anti-acetylcholine receptor (AChR) antibody-positive, anti-muscle-specific tyrosine kinase (MuSK) antibody-positive, or anti-low density lipoprotein receptor-related protein 4 (Lrp4) antibody-positive.

2. The pharmaceutical composition of claim 1, wherein the antibody is satralizumab.

3. The pharmaceutical composition of claim 1 or 2, wherein the patient with myasthenia gravis is anti-MuSK antibody-positive or anti-Lrp4 antibody-positive.

4. The pharmaceutical composition of any one of claims 1 to 3, wherein the patient with myasthenia gravis is a patient diagnosed with myasthenia gravis exhibiting generalized muscle weakness that falls under Class II, III, or IV of the Myasthenia Gravis Foundation of America (MGFA) Clinical Classification.

5. The pharmaceutical composition of any one of claims 1 to 3, wherein the patient with myasthenia gravis is a patient with a total MG Activities of Daily Living (MG-ADL) score of 5 or higher, and wherein half or more of the score is related to non-ocular symptoms.

6. The pharmaceutical composition of any one of claims 1 to 3, wherein the myasthenia gravis is generalized myasthenia gravis.

7. The pharmaceutical composition of any one of claims 1 to 3, which reduces a MG-ADL score.

8. The pharmaceutical composition of any one of claims 1 to 3, which reduces a Quantitative Myasthenia Gravis (QMG) score, 15-item Myasthenia Gravis (MG) Quality of Life scale (revised)(MG-QOL 15r) score, Neurology Quality-of-Life Fatigue Short Form (Neuro-QOL Fatigue) score, or Myasthenia Gravis Composite (MGC) score.

9. The pharmaceutical composition of any one of claims 1 to 8, wherein the dose of the antibody for a patient with a body weight of 100 kg or less is 120 mg/administration, and the dose of the antibody for a patient with a body weight of more than 100 kg is 180 mg/ administration.

10. The pharmaceutical composition of any one of claims 1 to 8, wherein the dose of the antibody for a patient with a body weight of 100 kg or less is 120 mg/administration, and the dose of the antibody for a patient with a body weight of more than 100 kg is 240 mg/administration.

11. The pharmaceutical composition of any one of claims 1 to 8, wherein the dose of the antibody for a patient with a body weight of 100 kg or less is 180 mg/administration, and the dose of the antibody for a patient with a body weight of more than 100 kg is 240 mg/administration.

12. The pharmaceutical composition of any one of claims 1 to 11, wherein the composition is administered at a standard dosing interval after a short-interval dosing period during which the composition is administered at the same dose as a standard dose multiple times at a dosing interval that is shorter than the standard dosing interval.
